# EUROPEAN PATENT APPLICATION

(11) **EP 3 184 543 A2**
(43) Date of publication of application: **28.06.2017**
(21) Application number: 16205331.8
(22) Date of filing: 20.12.2016
(51) Int. Cl.: C07K 16/26, C07K 16/22, A61K 39/00

(54) **IMMUNOPOTENTIATOR CONTAINING ANTI-ANG2 ANTIBODY**

(30) Priority: 24.12.2015 KR 20150186457
(71) Applicant: Samsung Electronics Co., Ltd, Gyeonggi-do 16677 (KR)
(72) Inventor: KANG, Yuhoi, Seongnam-si Gyeonggi-do (KR); LEE, Hojun, Seoul (KR); JO, Hongseok, Osan-si Gyeonggi-do (KR); PARK, Sang Chul, Seongnam-si Gyeonggi-do (KR); HAN, Sang Yeul, Yongin-si Gyeonggi-do (KR)
(74) Representative: Grünecker Patent- und Rechtsanwälte PartG mbB

(57) **Abstract**

Provided is a method of potentiating immunity or preventing and/or treating an immune-related disease comprising administering an anti-Ang2 antibody or an antigen-binding fragment thereof to a subject in need thereof.

## Description

### BACKGROUND OF THE INVENTION

### 1. Field of the Invention

Provided is a method of potentiating immunity or preventing and/or treating an immune-related disease comprising administering an anti-Ang2 antibody or an antigen-binding fragment thereof to a subject in need thereof.

### 2. Description of the Related Art

The attenuation of immune functions caused by senescence and various immune-related diseases (e.g., acquired immunodeficiency syndrome, autoimmune diseases, etc.) includes thymus involution, reduction in T cell proliferation, reduction in the response of T cells to foreign antigens, and an increase in immune response to autoantigens.

T cells are a type of immune cell that is involved in immunoregulation through the generation of cytokines responsible for immune cell proliferation and differentiation, and destruction of virus-infected cells, implanted cells, and tumor cells. T cells account for 60∼80% of peripheral blood lymphocytes, and can be distinguished from other lymphocytes by the presence of a T cell receptor (TCR) on the cell surface. The T cell receptor is responsible for recognizing fragments of antigens, and exists as a complex with CD2 and CD3. T cells are also divided into CD4+ T cells and CD8+ T cells according to the expression of CD4 or CD8. CD4+ T cells help the activity of other immune cells by releasing T cell cytokines, and can activate macrophages, which mediate delayed hypersensitivity. CD8+ T cells, known as cytotoxic T cells, that mainly function to destroy virus-infected cells and tumor cells. CD8+ T cells are also implicated in transplant rejection.

When T cell recognize an antigen, they actively proliferate and secret immunostimulants such as IL-2 cytokine, which is a protein that incites the immune system to defend against the invasion of germs. IL-2, produced from activated T cells, acts through IL-2 receptors in an intrathymic autocrine/paracrine pathway. Signaling through IL-2 receptors activates the Akt, MAP kinase, and JAK-STAT pathway, regulating biological functions associated with cell differentiation, growth, and survival.

Recent reports have suggested that the down-regulation of c-myc, c-jun, and c-fos genes (i.e., genes that play an important role in T cell activation) and AP-1 and NF-AT (i.e., transcription regulators of IL-2 cytokine) play a role in senescence. Furthermore, during the progression of senescence or HIV (Human Immunodeficiency Virus) infection, IL-2 expression is suppressed, so that the immune system cannot exhibit an effective response.

Conventional immunity-enhancing methods using small molecules are restricted because they are prone to non-specific binding, which leads to side effects. In addition, the treatment of AIDS is generally conducted with Highly Active Anti-Retroviral Therapy (HAART), which can reduce the level of the virus. However, HAART does not have any immunopotentiation effects. Alternatively, the reduction of inflammation with hormones, such as glucocorticoids, have been used to prevent immunity attenuation. However, these methods are limited in terms of regimen and dosage because the drugs react with target molecules, causing side effects such as cardiovascular diseases, cancer, osteoporosis.

Therefore there is a need for a therapy for alleviating the attenuation of immune function caused by senescence and/or immune-related diseases. This invention provides such a therapy.

### SUMMARY OF THE INVENTION

The present disclosure addresses a use of an anti-Ang2 antibody or an antigen-binding fragment thereof in immunopotentiation and/or in prevention and/or treatment of an immune-related disease.

An embodiment provides an immunopotentiator comprising an anti-Ang2 antibody or an antigen-binding fragment thereof.

Another embodiment provides an anti-Ang2 antibody or an antigen-binding fragment thereof for use in potentiating immunity or in preventing or treating an immune-related disease.

Another embodiment provides a method of potentiating immunity, comprising administering an anti-Ang2 antibody or an antigen-binding fragment thereof to a subject in need of potentiating immunity.

A further embodiment provides a pharmaceutical composition for use in preventing or treating an immune-related disease, comprising an anti-Ang2 antibody or an antigen-binding fragment thereof and a pharmaceutically acceptable carrier.

Still another embodiment provides a method of preventing and/or treating an immune-related disease, comprising administering an anti-Ang2 antibody or an antigen-binding fragment thereof to a subject in need of preventing or treating an immune-related disease.

The anti-Ang2 antibody or antigen-binding fragment thereof useful in the present disclosure may be characterized in that it binds specifically to Ang2 (angiopoietin-2) without interfering with the interaction between Ang2 and Tie2, thereby inhibiting Ang2, and/or inducing the activation of the Tie2 receptor.

### BRIEF DESCRIPTION OF THE DRAWINGS

The above and other objects, features and advantages of the present invention will be more clearly understood from the following detailed description taken in conjunction with the accompanying drawings, in which:
Fig. 1 is a photographic image showing a comparison in thymus size between subjects administered an anti-Ang2 antibody or a control antibody;
Fig. 2 is a graph in which the number of thymocytes is compared between subjects administered an anti-Ang2 antibody or a control antibody;
Fig. 3 is a graph in which the number of naive CD4+ T cells is compared between subjects administered an anti-Ang2 antibody or a control antibody;
Fig. 4 is a graph in which the percentage of IL-2-secreting CD4+ T cells is compared between subjects administered an anti-Ang2 antibody or a control antibody; and
Fig. 5 is a graph in which the percentage of self-renewal hematopoietic stem cells is compared between subjects administered an anti-Ang2 antibody or a control antibody.

### DETAILED DESCRIPTION

One embodiment provides embodiment provides an anti-Ang2 antibody or an antigen-binding fragment thereof for use in potentiating immunity. Another embodiment provides a pharmaceutical composition for use in potentiating immunity (immunopotentiator), comprising an anti-Ang2 antibody or an antigen-binding fragment thereof and optionally, a pharmaceutically acceptable carrier. Another embodiment provides a method for potentiating immunity, comprising administering an anti-Ang2 antibody or an antigen-binding fragment thereof to a subject in need thereof. The method may further comprise identifying (diagnosing or selecting) a subject that is in need of immunopotentiation prior to the administering step.

The immunopotentiation may refer to enhancement or increase in immunity, which may be exemplified by 1) improvement or increase in resistance to thymus involution, thymic function, the number of T cells, or production of hematopoietic stem cells, or 2) augmentation or recovery of T cell functions, and the like.

The immunopotentiation effect of the anti-Ang2 antibody or the antigen-binding fragment thereof was confirmed in anti-Ang2 antibody-administered senescent mice (24 months old) which were found to exhibit resistance to thymus involution (Examples 2 and 3), an improvement in thymic function and T cell count (Example 4), the augmentation (recovery) of T cell functions (Example 5), and a high level of hematopoietic stem cells (Example 6).

A further embodiment provides an anti-Ang2 antibody or an antigen-binding fragment thereof for use in preventing thymus involution and/or improving a thymic function. Another embodiment provides a pharmaceutical composition for use in preventing thymus involution and/or improving a thymic function, comprising an anti-Ang2 antibody or an antigen-binding fragment thereof, and optionally, a pharmaceutically acceptable carrier. A still further embodiment of the present invention is a method for preventing thymus involution and/or improving a thymic function, comprising administering an anti-Ang2 antibody or an antigen-binding fragment thereof to a subject in need thereof. The method may further comprise identifying (diagnosing or selecting) a subject that is in need of preventing thymus involution and/or improving a thymic function prior to the administering step. As used herein, the term "thymus involution" is intended to encompass, but is not limited to, the reduction of thymus size, thymocyte count, and/or T cell (e.g., CD4+ T cells) production function. Thymus involution may be caused by various factors comprising, but not limited to, senescence and/or immune diseases. The improvement of thymic functions means an improvement in thymus size, an increase in thymocyte count, and/or increased T cell production, but is not limited thereto.

Still another embodiment provides an anti-Ang2 antibody or an antigen-binding fragment thereof for use in augmenting T cells. A further embodiment provides a pharmaceutical composition for use in augmenting T cells, comprising an anti-Ang2 antibody or an antigen-binding fragment thereof, and optionally, a pharmaceutically acceptable carrier. Contemplated in accordance with yet another embodiment is a method for augmenting T cells, comprising administering an anti-Ang2 antibody or an antigen-binding fragment thereof to a subject in need thereof. The method may further comprise identifying (diagnosing or selecting) a subject that is in need of augmenting T cells prior to the administering step. The T cells may be a type of CD4 positive (CD4+) T cells. Here, the augmentation of T cells means the increase of a T cell count and/or the amelioration of T cell function (e.g., a higher expression level of cytokines, such as IL-2, interferon-γ, TNF-β, IL-10, etc), but is not limited thereto. In particular, because a suitable population level of CD4+ immune cells is strongly correlated with high survival rates and favorable prognosis for immune diseases such as acquired immune deficiency syndrome, an increased count of CD4+ T cells may be advantageous for immunopotentiation and treating various immune-related diseases, comprising acquired immune deficiency syndrome.

Yet a further embodiment provides an anti-Ang2 antibody or an antigen-binding fragment thereof for use in increasing the production of hematopoietic stem cells (self-renewal). A further embodiment provides a pharmaceutical composition for use in increasing the production of hematopoietic stem cells (self-renewal), comprising an anti-Ang2 antibody or an antigen-binding fragment thereof, and optionally, a pharmaceutically acceptable carrier. Another embodiment is a method for increasing the production of hematopoietic stem cells (self-renewal), comprising administering an anti-Ang2 antibody or an antigen-binding fragment thereof to a subject in need thereof. The method may further comprise identifying (diagnosing or selecting) a subject that is in need of increased production of hematopoietic stem cells (self-renewal) prior to the administering step. The hematopoietic stem cells may comprise, but are not limited to, self-renewing hematopoietic stem cells (e.g. long-term hematopoietic stem cells etc.).

An additional embodiment provides an anti-Ang2 antibody or an antigen-binding fragment thereof for use in preventing and/or treating an immune-related disease. A further embodiment provides a pharmaceutical composition for use in preventing and/or treating an immune-related disease, comprising an anti-Ang2 antibody or an antigen-binding fragment thereof, and optionally, a pharmaceutically acceptable carrier. Another additional embodiment provides a method for preventing and/or treating an immune-related disease, comprising administering an anti-Ang2 antibody or an antigen-binding fragment thereof to a subject in need thereof. The method may further comprise identifying (diagnosing or selecting) a subject that is in need of preventing or treating an immune-related disease, prior to the administering step. The immune-related disease may be at least one selected from the group consisting of acquired immune deficiency syndrome (AIDS) and autoimmune diseases (e.g., rheumatism, systemic lupus erythematosus, autoimmune hemolytic anemia, etc.).

The anti-Ang2 antibody or the antigen-binding fragment thereof is an antibody or an antigen-binding fragment thereof that specifically binds to Ang2, but does not inhibit binding between Ang2 and a Tie2 receptor. The antibody, thus, can form a complex with a Tie2 receptor through Ang2 (antibody-Ang2-Tie2 complex). The antibody or the antigen-binding fragment is characterized by dimerization, through which the Tie2 receptors of the complexes can be effectively clustered, thus inducing the activation of the Tie2 receptor and its downstream signaling. In such a mechanism of action, the antibody binds to Ang2 to induce the internalization and degradation of Ang2, thereby inhibiting Ang2 and lowering the level of circulating Ang2. By conjugating a Tie2 receptor together with Ang2 the antibody or the antigen-binding fragment thereof activates the Tie2 receptor in an 'Angl-like' manner to thus exhibit the dual function of inducing Tie2 downstream signaling and stabilizing vascular endothelial cells.

So long as it specifically recognize and binds to Ang2 and complexes with (coupled to) Tie2 through Ang2, any antibody or an antigen-binding fragment thereof may fall within the scope of the anti-Ang2 antibody or the antigen-binding fragment thereof in accordance with the present disclosure. Moreover, the anti-Ang2 antibody or the antigen-binding fragment thereof may induce the activation of a Tie2 receptor. Such Tie2 receptor activation may be triggered by phosphorylating a Tie2 receptor and/or a protein responsible for the downstream signal pathway thereof, for example, at least one protein selected from the group consisting Akt (NM_005163), eNOS (NM_000603), 42/44 (NM_002745), etc. Also, the anti-Ang2 antibody or the antigen-binding fragment thereof may induce the intracellular internalization of a Tie2 receptor. In other words, the anti-Ang2 antibody or the antigen-binding fragment thereof binds to Ang2 and induces the activation of a Tie2 receptor by forming a complex with a Tie2 receptor, together with Ang2, without inhibiting binding between Ang2 and the Tie2 receptor.An Ang2 protein, which acts as an antigen for the antibody provided by the present disclosure, is a soluble ligand present in the blood, functioning to promote tumor angiogenesis, metastasis, and invasion. Ang2 may be a protein originating from mammals comprising primates such as humans, monkeys, etc., and rodents such as mice, rats, etc. Examples of the Ang2 may comprise, but are not limited to, human Ang2 (e.g. NCBI Accession No. 015123), monkey Ang2 (e.g. NCBI Accession No. Q8MIK6), mouse Ang2 (e.g. NCBI Accession No. 035608), and rat Ang2 (e.g. NCBI Accession No. 035462).

The Tie2 receptor (TEK tyrosine kinase), which acts as an Angiopoietin-1 receptor, is expressed in vascular endothelial cells in various mammals such as mice (NM_013690; NP_038718), rats, and humans (NM_000459; NP_000450), and is involved in various downstream signaling pathways.

The anti-Ang2 antibody or the antigen-binding fragment thereof may recognize, as an epitope, full loop 1 (a region from 417^{th} to 434^{th} amino acid residue of SEQ ID NO: 11) of human Ang2 (hAng2; SEQ ID NO: 11; Accession #O15123) or a part thereof (e.g. at least one selected from the group consisting of externally exposed amino acid residues of the loop), or an amino acid sequence composed of 2 to 20, 2 to 15, 2 to 10, or 2 to 5 neighboring (consecutive) amino acids comprising at least one externally exposed amino acid residue of loop 1 of SEQ ID NO: 11, or may specifically bind thereto. As used herein, the term "externally exposed residue" refers to a residue that can be exposed to a biological medium (for example, a solution environment *in vivo,* e.g., physiological pH, temperature, isotonicity, etc.) to perform binding to a different protein.
Ang2 (SEQ ID NO: 11)

MWQIVFFTLS CDLVLAAAYN NFRKSMDSIG KKQYQVQHGS CSYTFLLPEM
DNCRSSSSPY VSNAVQRDAP LEYDDSVQRL QVLENIMENN TQWLMKLENY
IQDNMKKEMV EIQQNAVQNQ TAVMIEIGTN LLNQTAEQTR KLTDVEAQVL
NQTTRLELQL LEHSLSTNKL EKQILDQTSE INKLQDKNSF LEKKVLAMED
KHIIQLQSIK EEKDQLQVLV SKQNSIIEEL EKKIVTATVN NSVLQKQQHD
LMETVNNLLT MMSTSNSAKD PTVAKEEQIS FRDCAEVFKS GHTTNGIYTL
TFPNSTEEIK AYCDMEAGGG GWTIIQRRED GSVDFQRTWK EYKVGFGNPS
GEYWLGNEFV SQLTNQQRYV LKIHLKDWEG NEAYSLYEHF YLSSEELNYR
IHLKGLTGTA GKISSISQPG NDFSTKDGDN DKCICKCSQM LTGGWWFDAC
GPSNLNGMYY PQRQNTNKFN GIKWYYWKGS GYSLKATTMM IRPADF

By way of example, the anti-Ang2 antibody may recognize and bind, as an epitope, Q418, P419, a combination of Q418 and P419 positioned at loop 1 of SEQ ID NO: 11, or a region composed of 2 to 20, 2 to 15, 2 to 10, or 2 to 5 neighboring (consecutive) amino acids comprising Q418, P419 or a combination of Q418 and P419 on SEQ ID NO: 11. In one embodiment, the anti-Ang2 antibody may recognize and specifically bind the amino acid residues Q418 and P419 on SEQ ID NO: 11 as an epitope.

Q418, P419, and an amino acid region comprising them, which is an epitope for the anti-Ang2 antibody, are exposed amino acid residues positioned at loop 1 of the three-dimensional structure of Ang2, and are not implicated in binding between Ang2 and a Tie2 receptor.

As used in the context of Q418, P419, or an amino acid region comprising them, to which the anti-Ang2 antibody binds, the term "neighboring" (consecutive) amino acids may refer to amino acids which are adjacent to one another on a primary, secondary, or tertiary protein structure.

The antibody may be humanized or affinity matured.

In some embodiments, the anti-Ang2 antibody or an antigen-binding fragment thereof may comprise:
a heavy-chain complementarity-determining region (CDR) comprising at least one selected from the group consisting of a polypeptide comprising the amino acid sequence of SEQ ID NO: 1 (CDR-H1), a polypeptide comprising the amino acid sequence of SEQ ID NO: 2 (CDR-H2), and a polypeptide comprising the amino acid sequence of SEQ ID NO: 3 (CDR-H3), or a heavy-chain variable region comprising the heavy-chain complementarity-determining region;
a light-chain complementarity-determining region selected from the group consisting of a polypeptide comprising the amino acid sequence of SEQ ID NO: 4 (CDR-L1), a polypeptide comprising the amino acid sequence of SEQ ID NO: 5 (CDR-L2), and a polypeptide comprising the amino acid sequence of SEQ ID NO: 6 (CDR-L3), or a light chain variable region comprising the light-chain complementarity-determining region;
a combination of the at least one heavy-chain complementarity-determining region and the at least one light-chain complementarity-determining region; or
a combination of the heavy-chain variable region and the light chain variable region.

According to a particular embodiment, the anti-Ang2 antibody or an antigen-binding fragment thereof may comprise, consist essentially of, or consist of:
a heavy-chain complementarity-determining region comprising a polypeptide comprising the amino acid sequence of SEQ ID NO: 1 (CDR-H1), a polypeptide comprising the amino acid sequence of SEQ ID NO: 2 (CDR-H2), and a polypeptide comprising the amino acid sequence of SEQ ID NO: 3 (CDR-H3), or a heavy-chain variable region comprising the at least one heavy-chain complementarity-determining region; and
a light-chain complementarity-determining region comprising a polypeptide comprising the amino acid sequence of SEQ ID NO: 4 (CDR-L1), a polypeptide comprising the amino acid sequence of SEQ ID NO: 5 (CDR-L2), and a polypeptide comprising the amino acid sequence of SEQ ID NO: 6 (CDR-L3), or a light chain variable region comprising the light-chain complementarity-determining region.

In one embodiment, the heavy-chain variable region of the anti-Ang2 antibody or the antigen-binding fragment may comprise, consist essentially of, or consist of the amino acid sequence of SEQ ID NO: 7:

DVQLQESGPDLVKPSQSLSLTCTVTGYSITSDYAWNWIRQFPGNKLEW
MGYINYSGNTDYNPSLKSRSSITRDTSKNQFFLQLNSVTTGDTATYYCARGN

FEGAMDYWGQGTLVTVSS (SEQ ID NO: 7)

(In SEQ ID NO: 7, the underlined bold letters represent CDR-H1, CDR-H2, and CDR-H3 in sequence)

The light chain variable region of the antibody according to one embodiment may comprise the amino acid sequence of SEQ ID NO: 9:

SIVMTQTPKFLLVSAGDRVTITCKASQSVSNDVAWYQQKPGQSPKLLI
YYASNRYPGVPDRFTGSGYGTDFTFTISTVQAEDLAVYFCQQDYSSPWTFGG
GTKLEIK(SEQ ID NO: 9)

(In SEQ ID NO: 9, the underlined bold letters represent CDR-L1, CDR-L2, and CDR-L3 in sequence)

Accordingly, the anti-Ang2 antibody or an antigen-binding fragment thereof may comprise, consist essentially of, or consist of a heavy-chain variable region comprising the amino acid sequence of SEQ ID NO: 7, a light chain variable region comprising the amino acid sequence of SEQ ID NO: 9, or a combination of the heavy-chain variable region and the light chain variable region.

For example, the anti-Ang2 antibody or an antigen-binding fragment thereof may comprise, consist essentially of, or consist of a heavy-chain variable region comprising the amino acid sequence of SEQ ID NO: 7, and a light chain variable region comprising the amino acid sequence of SEQ ID NO: 9.

In some embodiments, the anti-Ang2 antibody or an antigen-binding fragment thereof may be provided with enhanced affinity for Ang2 by partial substitution on the amino acid sequence of at least one CDR, for example, at least one selected from among CDR-H2, CDR-L1, and CFR-L3, while maintaining the intrinsic antibody activity.

According to an embodiment, the affinity-enhanced (affinity-matured) anti-Ang2 antibody or an antigen-binding fragment thereof may comprise at least one substitution selected from the group consisting of:
(1) substitution of the first amino acid residue Tyr (Y) on the amino acid sequence (YINYSGNTDYNPSLKS; SEQ ID NO: 2) of CDR-H2 with Lys (K);
(2) substitution of the third amino acid residue Asn (N) on the amino acid sequence (YINYSGNTDYNPSLKS; SEQ ID NO: 2) of CDR-H2 with Ser (S);
(3) substitution of the fifth amino acid residue Ser (S) on the amino acid sequence (YINYSGNTDYNPSLKS; SEQ ID NO: 2) of CDR-H2 with Ala (A);
(4) substitution of the seventh amino acid residue Asn (N) on the amino acid sequence (YINYSGNTDYNPSLKS; SEQ ID NO: 2) of CDR-H2 with Lys (K);
(5) substitution of the eleventh (last) amino acid residue Ala (A) on the amino acid sequence (KASQSVSNDVA; SEQ ID NO: 4) of CDR-L1 with His (H);
(6) substitution of the fifth amino acid residue Ser (S) on the amino acid sequence (KASQSVSNDVA; SEQ ID NO: 4) of CDR-L1 with Phe (F);
(7) substitution of the eighth amino acid residue Asn (N) on the amino acid sequence (KASQSVSNDVA; SEQ ID NO: 4) of CDR-L1 with Thr (T)
(8) substitution of the fourth amino acid residue Asn (N) on the amino acid sequence (YASNRYP; SEQ ID NO: 5) of CDR-L2 with Ile (I);
(9) substitution of the fifth amino acid residue Arg (R) on the amino acid sequence (YASNRYP; SEQ ID NO: 5) of CDR-L2 with Pro (P);
(10) substitution of the second amino acid residue Gln (Q) on the amino acid sequence (QQDYSSPWT; SEQ ID NO: 6) of CDR-L3 with His (H); and
(11) substitution of the eight amino acid residue Trp (W) on the amino acid sequence (QQDYSSPWT; SEQ ID NO: 6) of CDR-L3 with Phe (F).

In some embodiments, the affinity-enhanced anti-Ang2 antibody or an antigen-binding fragment thereof may comprise an amino acid sequence of Formula 1 (SEQ ID NO: 20), as a CDR-H2:
X1-I-X2-Y-X3-G-X4-T-D-Y-N-P-S-L-K-S (Formula 1: SEQ ID NO: 20)
wherein, X1 is Tyr (Y) or Lys (K), X2 is Asn (N) or Ser (S), X3 is Ser (S) or Ala (A), and X4 is Asn (N) or Lys (K).

According to another embodiment, the amino acid sequence of SEQ ID NO: 20 may be SEQ ID NO: 14 or SEQ ID NO: 15.

In some embodiments, the affinity-enhanced anti-Ang2 antibody and an antigen-binding fragment thereof may comprise an amino acid sequence of Formula 2 (SEQ ID NO: 21), as a CDR-L1:
K-A-S-Q-X5-V-S-X6-D-V-X7 (Formula 2: SEQ ID NO: 21)
wherein, X5 is Ser (S) or Phe (F), X6 is Asn (N) or Thr (T), and X7 is Ala (A) or His (H).

In a particular embodiment, the amino acid sequence of SEQ ID NO: 21 may be SEQ ID NO: 16 or SEQ ID NO: 17.

In some embodiments, the affinity-enhanced anti-Ang2 antibody and an antigen-binding fragment thereof may comprise an amino acid sequence of Formula 3 (SEQ ID NO: 22), as a CDR-L2:
Y-A-S-X8-X9-Y-P (Formula 3: SEQ ID NO: 22)
wherein, X8 is Asn (N) or Ile (I), and X9 is Arg (R) or Pro (P).

According to a particular embodiment, the amino acid sequence of SEQ ID NO: 22 may be SEQ ID NO: 18.

In some embodiments, the affinity-enhanced anti-Ang2 antibody and an antigen-binding fragment thereof may comprise an amino acid sequence of Formula 4 (SEQ ID NO: 23), as a CDR-L3:
Q-X10-D-Y-S-S-P-X11-T (Formula 4: SEQ ID NO: 23)
wherein, X10 is Gln (Q) or His (H), and X11 is Trp (W) or Phe (F).

According to a particular embodiment, the amino acid sequence of SEQ ID NO: 23 may be identical to that of SEQ ID NO: 19.

Accordingly, the affinity-enhanced anti-Ang2 antibody or an antigen-binding fragment thereof may comprise, consist essentially of, or consist of:
heavy-chain complementarity-determining regions (CDRs) comprising:
   a polypeptide comprising the amino acid sequence of SEQ ID NO: 1 (CDR-H1),
   a polypeptide comprising the amino acid sequence of SEQ ID NO: 2 (CDR-H2), and
   a polypeptide comprising the amino acid sequence of SEQ ID NO: 3 (CDR-H3), or
a heavy-chain variable region comprising the heavy-chain complementarity-determining regions;
light-chain complementarity-determining regions comprising:
   a polypeptide comprising the amino acid sequence of SEQ ID NO: 21 (CDR-L1),
   a polypeptide comprising the amino acid sequence of SEQ ID NO: 22 (CDR-L2), and
   a polypeptide comprising the amino acid sequence of SEQ ID NO: 23 (CDR-L3), or
a light chain variable region comprising the light-chain complementarity-determining regions;
a combination of the heavy-chain complementarity-determining regions and the light-chain complementarity-determining regions; or
a combination of the heavy-chain variable region and the light chain variable region.

In a particular embodiment, the affinity-enhanced anti-Ang2 antibody or an antigen-binding fragment thereof may comprise:
heavy-chain complementarity-determining regions comprising:
   a polypeptide comprising the amino acid sequence of SEQ ID NO: 1 (CDR-H1),
   a polypeptide comprising an amino acid sequence selected from the group consisting of SEQ ID NO: 2, SEQ ID NO: 14, and SEQ ID NO: 15 (CDR-H2), and
   a polypeptide comprising the amino acid sequence of SEQ ID NO: 3 (CDR-H3), or
a heavy-chain variable region comprising the heavy-chain complementarity-determining regions;
light-chain complementarity-determining regions comprising:
   a polypeptide comprising the amino acid sequence of SEQ ID NO: 4, SEQ ID NO: 16, or SEQ ID NO: 17 (CDR-L1),
   a polypeptide comprising the amino acid sequence of SEQ ID NO: 5 or SEQ ID NO: 18 (CDR-L2), and
   a polypeptide comprising the amino acid sequence of SEQ ID NO: 6 or SEQ ID NO: 19, or
a light chain variable region comprising the light-chain complementarity-determining regions;
a combination of the heavy-chain complementarity-determining regions and the light-chain complementarity-determining regions; or
a combination of the heavy-chain variable region and the light-chain variable region.

In an embodiment, for the purpose of being distinguished from the template anti-Ang2-antibody or antibody fragment, the affinity-enhanced anti-Ang2 antibody or an antigen-binding fragment does not comprise all of a CDR-H1 comprising the amino acid sequence of SEQ ID NO: 1, a CDR-H2 comprising the amino acid sequence of SEQ ID NO: 2, a CDR-H3 comprising the amino acid sequence of SEQ ID NO: 3a CDR-L1 comprising the amino acid sequence of SEQ ID NO: 4, a CDR-L2 comprising the amino acid sequence of SEQ ID NO: 5, and a CDR-L3 comprising the amino acid sequence of SEQ ID NO: 6.

The heavy-chain complementarity-determining regions and light-chain complementarity-determining regions of the above-described templates and affinity-enhanced anti-Ang2 antibodies or their antigen-binding fragments are summarized in Table 1.

**TABLE 1**

| Amino acid sequence of heavy chain CDR | | | |
|---|---|---|---|
| | CDRH1-KABAT | CDRH2-KABAT | CDRH3-KABAT |
| Template | SDYAWN(SEQ ID NO:1) | YINYSGNTDYNPSLKS(SE Q ID NO: 2) | GNFEGAMDY(SEQ ID NO: 3) |
| Affinity-matured | - | KISYSGKTDYNPSLKS(SE Q ID NO: 14) | - |
| | - | KINYAGNTDYNPSLKS(S EQ ID NO: 15) | - |

| Amino acid sequence of light chain CDR | | | |
|---|---|---|---|
| | CDRL1-KABAT | CDRL2-KABAT | CDRL3-KABAT |
| Template | KASQSVSNDVA (SEQ ID NO.4) | YASNRYP (SEQ ID NO: 5) | QQDYSSPWT (SEQ ID NO: 6) |
| Affinity-matured | KASQSVSNDVH(SEQ ID NO: 16) | YASIPYP(SEQ ID NO: 18) | QHDYSSPFT(SEQ ID NO: 19) |
| | KASQFVSTDVH(SEQ ID NO: 17) | | |

In some embodiments, the affinity-enhanced anti-Ang2 antibody or an antigen-binding fragment thereof may be selected from the group consisting of:
(a) an antibody or an antigen-binding fragment thereof comprising:
   a heavy-chain complementarity-determining region comprising the CDR-H1 of SEQ ID NO: 1, the CDR-H2 of SEQ ID NO: 14, and the CDR-H3 of SEQ ID NO: 3, or a heavy-chain variable region comprising the heavy-chain complementarity-determining region; and
   a light-chain complementarity-determining region comprising the CDR-L1 of SEQ ID NO: 4, the CDR-L2 of SEQ ID NO: 5, the CDR-L3 of SEQ ID NO: 6, or a light chain variable region comprising the light-chain complementarity-determining region;
(b) an antibody or an antigen-binding fragment thereof comprising:
   a heavy-chain complementarity-determining region comprising the CDR-H1 of SEQ ID NO: 1, the CDR-H2 of SEQ ID NO: 14, and the CDR-H3 of SEQ ID NO: 3, or a heavy-chain variable region comprising the heavy-chain complementarity-determining region; and
   a light-chain complementarity-determining region comprising the CDR-L1 of SEQ ID NO: 16, the CDR-L2 of SEQ ID NO: 5, and the CDR-L3 of SEQ ID NO: 6; or a light chain variable region comprising the light-chain complementarity-determining region;
(c) an antibody or an antigen-binding fragment thereof comprising:
   a heavy-chain complementarity-determining region comprising the CDR-H 1 of SEQ ID NO: 1, the CDR-H2 of SEQ ID NO: 15, and the CDR-H3 of SEQ ID NO: 3, or a heavy-chain variable region comprising the heavy-chain complementarity-determining region; and
   a light-chain complementarity-determining region comprising the CDR-L1 of SEQ ID NO: 4, the CDR-L2 of SEQ ID NO: 5, and the CDR-L3 of SEQ ID NO: 6, or a light chain variable region comprising the light-chain complementarity-determining region;
(d) an antibody or an antigen-binding fragment thereof comprising:
   a heavy-chain complementarity-determining region comprising the CDR-H 1 of SEQ ID NO: 1, the CDR-H2 of SEQ ID NO: 15, and the CDR-H3 of SEQ ID NO: 3, or a heavy-chain variable region comprising the heavy-chain complementarity-determining region; and
   a light-chain complementarity-determining region comprising the CDR-L1 of SEQ ID NO: 16, the CDR-L2 of SEQ ID NO: 5, and the CDR-L3 of SEQ ID NO: 6, or a light chain variable region comprising the light-chain complementarity-determining region;
(e) an antibody or an antigen-binding fragment thereof comprising:
   a heavy-chain complementarity-determining region comprising the CDR-H 1 of SEQ ID NO: 1, the CDR-H2 of SEQ ID NO: 2, and the CDR-H3 of SEQ ID NO: 3, or heavy-chain variable region comprising the heavy-chain complementarity-determining region; and
   a light-chain complementarity-determining region comprising the CDRL1 of SEQ ID NO: 16, the CDR-L2 of SEQ ID NO: 5, and the CDR-L3 of SEQ ID NO: 19, or a light chain variable region comprising the light-chain complementarity-determining region;
(f) an antibody or an antigen-binding fragment thereof comprising:
   a heavy-chain complementarity-determining region comprising the CDR-H1 of SEQ ID NO: 1, the CDR-H2 of SEQ ID NO: 14, and the CDR-H3 of SEQ ID NO: 3, or a heavy-chain variable region comprising the heavy-chain complementarity-determining region; and
   a light-chain complementarity-determining region comprising the CDRL1 of SEQ ID NO: 17, the CDR-L2 of SEQ ID NO: 5, and the CDR-L3 of SEQ ID NO: 6, or a light chain variable region comprising the light-chain complementarity-determining region;
(g) an antibody or an antigen-binding fragment thereof comprising:
   a heavy-chain complementarity-determining region comprising the CDR-H1 of SEQ ID NO: 1, the CDR-H2 of SEQ ID NO: 14, and the CDR-H3 of SEQ ID NO: 3, or a heavy-chain variable region comprising the heavy-chain complementarity-determining region; and
   a light-chain complementarity-determining region comprising the CDR-L1 of SEQ ID NO: 4, the CDR-L2 of SEQ ID NO: 18, and the CDR-L3 of SEQ ID NO: 6, or a light chain variable region comprising the light-chain complementarity-determining region;
(h) an antibody or an antigen-binding fragment thereof comprising:
   a heavy-chain complementarity-determining region comprising the CDR-H1 of SEQ ID NO: 1, the CDR-H2 of SEQ ID NO: 14, and the CDR-H3 of SEQ ID NO: 3, or a heavy-chain variable region comprising the heavy-chain complementarity-determining region; and
   a light-chain complementarity-determining region comprising the CDR-L1 of SEQ ID NO: 16, the CDR-L2 of SEQ ID NO: 18, and the CDR-L3 of SEQ ID NO: 6, or a light chain variable region comprising the light-chain complementarity-determining region; and
(i) an antibody or an antigen-binding fragment thereof comprising:
   a heavy-chain complementarity-determining region comprising the CDR-H1 of SEQ ID NO: 1, the CDR-H2 of SEQ ID NO: 14, and the CDR-H3 of SEQ ID NO: 3, or a heavy-chain variable region comprising the heavy-chain complementarity-determining region; and
   a light-chain complementarity-determining region comprising the CDR-L1 of SEQ ID NO: 17, the CDR-L2 of SEQ ID NO: 18, and the CDR-L3 of SEQ ID NO: 6, or a light chain variable region comprising the light-chain complementarity-determining region.

The affinity-enhanced anti-Ang2 antibody or an antigen-binding fragment thereof may have an affinity (KD) of 10 nM or less, 5 nM or less, 2 nM or less, or 1 nM or less, for example, 0.01 to 10 nM, 0.01 to 5 nM, 0.01 to 2 nM, or 0.01 to 1 nM for Ang2. This affinity is significantly increased compared to the template anti-Ang2 antibody, showing an affinity (KD) of about 8 nM for Ang2.

Another embodiment addresses a humanized anti-Ang2 antibody or an antigen-binding fragment thereof. The humanized anti-Ang2 antibody or antigen-binding fragment thereof can be constructed by substituting some of the amino acid residues on the framework other than the complementarity-determining region of the heavy-chain variable region (SEQ ID NO: 7). The amino acid sequences of the heavy-chain framework available for the construction of such a humanized anti-Ang2 antibody or antigen-binding fragment thereof are listed in Table 2, below.

**TABLE 2**

| (Humanization of heavy chain) | | | | |
|---|---|---|---|---|
| | FR1 (framework adjacent to N-terminus of CDR-H1) | FR2 (framework between CDR-H1 and CDR-H2) | FR3 (framework between CDR-H2 and CDR-H3) | FR4 (framework adjacent to C-terminus of CDR-H3) |
| Frameworks in the heavy chain variable region of the template (SEQ ID NO: 7) | DVQLQESGPDLV KPSQSLSLTCTVT GYSIT(SEQ ID NO: 24) | WIRQFPGNKLEW MG(SEQ ID NO: 29) | RSSITRDTSKNQF FLQLNSVTTGDT ATYYCAR(SEQ ID NO: 34) | WGQGTLVTVSS( SEQ ID NO: 39) |
| Frameworks in a heavy chain variable region of a humanized antibody (VH-hu1) | QVQLQESGPGLV KPSETLSLTCAVS GYSIS(SEQ ID NO: 25) | WIRQPPGKGLEW IG(SEQ ID NO: 30) | RVTISVDTSKNQ FSLKLSSVTAAD TAVYYCAR(SEQ ID NO: 35) | WGQGTLVTVSS( SEQ ID NO: 40) |
| Frameworks in a heavy chain variable region of a humanized antibody (VH-hu2) | QVQLQESGPGLV KPSETLSLTCAVS GYSIT(SEQ ID NO: 26) | WIRQPPGKGLEW MG(SEQ ID NO: 31) | RSTISRDTSKNQF SLKLSSVTAADT AVYYCAR(SEQ ID NO: 36) | WGQGTLVTVSS( SEQ ID NO: 41) |
| Frameworks in a heavy chain variable region of a humanized antibody (VH-hu5) | QVQLQESGPGLV KPSETLSLTCAVS GYSIT(SEQ ID NO: 27) | WIRQPPGKGLEW IG(SEQ ID NO: 32) | RVTISVDTSKNQ FSLKLSSVTAAD TAVYYCAR(SEQ ID NO: 37) | WGQGTL VTVSS( SEQ ID NO: 42) |
| Frameworks in a heavy chain variable region of a humanized antibody (VH-hu3) | EVQLVESGGGLV QPGGSLRLSCAA SGYSIT(SEQ ID NO: 28) | WVRQAPGKGLE WMG(SEQ ID NO: 33) | RSTISRDTSKNTF YLQMNSLRAED TAVYYCAR(SEQ ID NO: 38) | WGQGTLVTVSS( SEQ ID NO: 43) |

In addition, the humanized anti-Ang2 antibody or antigen-binding fragment thereof can be constructed by substituting some of the amino acid residues on the framework other than the complementarity-determining region of the light-chain variable region (SEQ ID NO: 9). Amino acid sequences of the light-chain framework available for the construction of the humanized anti-Ang2 antibody or an antigen-binding fragment thereof are listed in Table 3, below.

**TABLE 3**

| (Humanization of light chain) | | | | |
|---|---|---|---|---|
| | FR1 (framework adjacent to N-terminus of CDR-L1) | FR2 (framework between CDR-L1 and CDR-L2) | FR3 (framework between CDR-L2 and CDR-L3) | FR4 (framework adjacent to C-terminus of CDR-L3) |
| Frameworks in the light chain variable region of the template (SEQ ID NO: 9) | SIVMTQTPKFLLV SAGDRVTITC(SE Q ID NO: 44) | WYQQKPGQSPKL LIY(SEQ ID NO: 46) | GVPDRFTGSGYG TDFTFTISTVQAE DLAVYFC(SEQ ID NO: 48) | FGGGTKLEIK(SE Q ID NO: 50) |
| Frameworks in a light chain variable region of a humanized antibody (VL-hul) | DIQMTQSPSSLSA SVGDRVTITC(SE Q ID NO: 45) | WYQQKPGKAPKL LIY(SEQ ID NO: 47) | GVPSRFSGSGSGT DFTLTISSLQPEDF ATYYC(SEQ ID NO: 49) | FGQGTKVEIK(SE Q ID NO: 51) |

In accordance with a specific embodiment thereof, the present disclosure addresses a humanized anti-Ang2 antibody comprising a heavy-chain variable region, a light-chain variable region, or both thereof, or an antigen-binding fragment thereof, wherein
the heavy-chain variable region comprises:
a polypeptide comprising an amino acid sequence selected from the group consisting of SEQ ID NO: 24, SEQ ID NO: 25, SEQ ID NO: 26, SEQ ID NO: 27, or SEQ ID NO: 28, for example, SEQ ID NO: 25 to SEQ ID NO: 28, as an N-terminal framework region of CDR-H1 (framework adjacent to N-terminus of CDR-H1);
a polypeptide comprising an amino acid sequence selected from the group consisting of SEQ ID NO: 29, SEQ ID NO: 30, SEQ ID NO: 31, SEQ ID NO: 32, or SEQ ID NO: 33. For example, SEQ ID NO: 30 to SEQ ID NO: 33, as a framework region between CDR-H1 and CDR-H2;
a polypeptide comprising an amino acid sequence selected from the group consisting of SEQ ID NO: 34, SEQ ID NO: 35, SEQ ID NO: 36, SEQ ID NO: 37, or SEQ ID NO: 38. For example, SEQ ID NO: 35 to SEQ ID NO: 38, as a framework region between CDR-H2 and CDR-H3, and
a polypeptide comprising an amino acid sequence selected from the group consisting of SEQ ID NO: 39, SEQ ID NO: 40, SEQ ID NO: 41, SEQ ID NO: 42, or SEQ ID NO: 43, for example, SEQ ID NO: 40 to SEQ ID NO: 43, as a C-terminal framework region of CDR-H3 (framework adjacent to C-terminus of CDR-H3); and
the light-chain variable region comprises:
a polypeptide comprising the amino acid sequence of SEQ ID NO: 44 or SEQ ID NO: 45, for example, the amino acid sequence of SEQ ID NO: 45, as an N-terminal framework region of CDR-L1 (framework adjacent to N-terminus of CDR-L1),
a polypeptide comprising the amino acid sequence of SEQ ID NO: 46 or SEQ ID NO: 47, for example, the amino acid sequence of SEQ ID NO: 47, as a framework region between CDR-L1 and CDR-L2,
a polypeptide comprising the amino acid sequence of SEQ ID NO: 48 or SEQ ID NO: 49, for example, the amino acid of SEQ ID NO: 49, as a framework region between CDR-L2 and CDR-L3, and
a polypeptide comprising the amino acid sequence of SEQ ID NO: 50 or SEQ ID NO: 51, for example, the amino acid sequence of SEQ ID NO: 51, as a C-terminal framework region of CDR-L3 (framework adjacent to C-terminus of CDR-L3).

In an embodiment, for the purpose of being distinguished from the template anti-Ang2 antibody or antigen-binding fragment thereof, the humanized anti-Ang2 antibody or an antigen-binding fragment thereof is not an antibody or antibody fragment in which the heavy-chain variable region comprises the amino acid sequence of SEQ ID NO: 24 as an N-terminal framework region of CDR-H1, the amino acid sequence of SEQ ID NO: 29 as a framework region between CDR-H1 and CDR-H2, the amino acid sequence of SEQ ID NO: 34 as a framework region between CDR-H2 and CDR-H3, and the amino acid sequence of CDR-H3 as a C-terminal framework region; and the light-chain variable region comprises the amino acid sequence of SEQ ID NO: 44 as an N-terminal framework region of CDR-L1, the amino acid sequence of SEQ ID NO: 46 as a framework region between CDR-L1 and CDR-L2, the amino acid sequence of SEQ ID NO: 48 as a framework region between CDR-L2 and CDR-L3, and the amino acid sequence of SEQ ID NO: 50 as a C-terminal framework region of CDR-L3.

In a particular embodiment, the humanized anti-Ang2 antibody or antigen-binding fragment thereof may comprise a heavy-chain variable region comprising an amino acid sequence selected from the group consisting of SEQ ID NO: 52, SEQ ID NO: 53, SEQ ID NO: 54, SEQ ID NO: 55, and SEQ ID NO: 56, and a light-chain variable region comprising an amino acid sequence selected from the group consisting of SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 87, and SEQ ID NO: 89.

The anti-Ang2 antibody useful in the present disclosure may be an anti-Ang2 antibody produced by the hybridoma cell line of Accession No. KCLRF-BP-00295, or may be an affinity-enhanced and/or humanized antibody derived therefrom.

Also, all the anti-Ang2 antibodies or their antigen-binding fragments described in Korean Patent Unexamined Application Publication No. 2015-0136031, which is hereby incorporated in its entirety by reference, fall within the scope of the present disclosure.

An animal-derived antibody that is produced by immunizing an animal with a desired antigen may generally trigger an immune rejection response when administered to humans for treatment purposes, and thus a chimeric antibody has been developed to suppress such immune rejection response. A chimeric antibody is formed by replacing the constant region of an animal-derived antibody, which is the cause of such an anti-isotype response, with that of a human antibody, using a genetic engineering method. Although the chimeric antibody is considerably mitigated in anti-isotype response in comparison with animal-derived antibodies, animal-derived amino acids present in the variable regions still retain the potential for side effects resulting from an anti-idiotypic response. Therefore, a humanized antibody has been developed to further mitigate such side effects. This can be constructed by grafting CDR (complementarity determining regions), which, of the variable regions of a chimeric antibody, play an important role in antigen binding, into a human antibody framework.

According to one embodiment, the antibody may be an animal-derived antibody (e.g., a mouse-derived antibody, etc.), a chimeric antibody (e.g., a mouse-human chimeric antibody, etc.), a humanized antibody, or a human antibody. The antibody or antigen-binding fragment thereof may be isolated from a living body or may be a non-naturally occurring substance. In this case, the antibody or antigen-binding fragment thereof may be recombinant or synthetic.

Since antibodies have a long half-life with high stability *in vivo* as well as *in vitro,* they are advantageous for mass expression and production. Also, an antibody, the structure of which is intrinsically dimeric, has fairly high avidity.An intact antibody has a structure composed of two full-length light chains and two full-length heavy chains, with linkages between the light chains and the heavy chains via disulfide bonds. The constant region of an antibody is divided into a heavy chain constant region and a light chain constant region. The heavy chain constant region has gamma (γ), mu (µ), alpha (α), delta (δ) and epsilon (ε) types, and has gamma (γ1), gamma2 (γ2), gamma3 (γ3), gamma4 (γ4), alpha1 (α1) and alpha2 (α2) as its subclasses. The light chain constant region has kappa (κ) and lambda (λ) types.

As used herein, the term "heavy chain" is intended to encompass a full-length heavy chain, which consists of a variable region domain V_{H} comprising an amino acid sequence having variable region sequences sufficient to provide specificity for antigen binding, three constant region domains C_{H1}, C_{H2} and C_{H3} domains, and a hinge, and a fragment thereof. The term "light chain" is understood to encompass a full-length light chain, which consists of a variable region domain V_{L} comprising an amino acid sequence having variable region sequences sufficient to contribute to specificity for antigen binding, a constant region domain C_{L}, and a fragment thereof.

The term "CDR (Complementarity Determining Region)" refers to an amino acid sequence found in the hypervariable region of a heavy chain and a light chain of an immunoglobulin. The heavy and light chain each comprise three CDRs (CDRH1, CDRH2, CDRH3, and CDRL1, CDRL2, CDRL3). The CDRs of an antibody can provide an essential contact residue for binding to an antigen or an epitope.

Throughout the specification, the terms "specifically binding" or "specifically recognizing" has the same meaning as generally known to a person of ordinary skill in the art, indicating that an antigen and an antibody specifically interact with each other to cause an immunological response.

The antigen-binding fragment of an antibody, provided by the present disclosure, may be a fragment comprising at least one complementarity determining region.

The term "antigen-binding fragment," means a fragment of the full structure of an immunoglobulin, which is a partial polypeptide comprising a domain to which an antigen can bind. For example, it may be scFv, (scFv)₂, scFv-Fc, Fab, Fab', or F(ab')₂, but is not limited thereto.

Of the antigen-binding fragments, a Fab fragment is characterized by a structure composed of one variable and one constant domain from the light chain, and one variable and the first constant (C_{H1}) domain from the heavy chain, retaining one paratope. A Fab' fragment is different from Fab in that Fab' further comprises a hinge region having at least one cysteine residue at the C-terminus of the heavy chain C_{H1} domain. A F(ab')₂ fragment forms as two Fab' fragments are joined by a disulfide bond between the cysteine residues of the hinge region. An Fv fragment is a minimal antibody fragment, having only heavy chain variable regions and light chain variable regions, and recombinant techniques for producing the Fv fragment are well known in the art. In a two-chain Fv fragment, the heavy chain variable domains are associated with the light chain variable domains via a non-covalent bond. A single-chain Fv fragment has a structure in which a heavy chain variable domain and a light chain variable domain are covalently joined to each other, either via a covalent bond or directly at the C-terminus, so that it can form a dimer, as in a two-chain Fv fragment. In this context, the heavy chain variable region and the light chain variable region may be connected with each other, either through a linker such as a peptide linker or directly. The peptide linker may be composed of 1 to 100 amino acid residues or, more preferably, 2 to 50 amino acid residues. Amino acid sequences suitable for use in the peptide linker may be those well known in the art. The antigen-binding fragments may be produced using proteases (for example, a complete antibody can be restrictedly digested into Fab with papain and into F(ab')₂ with pepsin) or a recombinant DNA technique.

Except for its CDR or variable regions, the anti-Ang2 antibody or an antigen-binding fragment thereof may be derived from the constant region of a human antibody. For example, the constant region of the antib-Ang2 antibody can be derived from IgA, IgE, or IgG, e.g., IgG1, IgG2, IgG 3, or IgG4.

The anti-Ang2 antibody may be monoclonal or polyclonal. Monoclonal antibodies may be prepared using a method known in the art, for example, a phage display technique. Alternately, the anti-Ang2 antibody may be prepared into a mouse-derived monoclonal antibody by methods set forth in Schwaber et al. (Schwaber, J and Cohen, E. P., "Human x Mouse Somatic Cell Hybrid Clones Secreting Immunoglobulins of Both Parental Types," Nature, 244 (1973), 444-447).

Meanwhile, individual monoclonal antibodies may be screened using a typical ELISA (Enzyme-Linked ImmunoSorbent Assay) format, based on the binding potential with Ang2. Inhibitory activity can be verified through functional analysis such as competitive ELISA for verifying the molecular interaction of binding assemblies or functional analysis such as a cell-based assay. Then, monoclonal antibody members selected on the basis of their strong inhibitory activities may each be verified for their affinities (K_{d} values) to Ang2.

In the method, the subject to which the antibody is administered may be a mammal, examples of which comprise primates such as humans or a monkeys, and rodents such as rats and mice, or may be a biological sample isolated from mammals or artificially cultured, such as cells, tissues, body fluids, etc.

The anti-Ang2 antibody or an antigen-binding fragment thereof may be used as an active ingredient in a pharmaceutical composition. The pharmaceutical composition may comprise a pharmaceutically acceptable carrier. Examples of the pharmaceutically acceptable carrier available for the pharmaceutical composition of the present disclosure comprise lactose, dextrose, sucrose, sorbitol, mannitol, starch, gum acacia, calcium phosphate, alginate, gelatin, calcium silicate, micro-crystalline cellulose, polyvinylpyrrolidone, cellulose, water, syrup, methyl cellulose, methylhydroxy benzoate, propylhydroxy benzoate, talc, magnesium stearate, and mineral oil, but are not limited thereto. Further, the pharmaceutical composition may comprise a typical additive selected from the group consisting of a diluent, an excipient, a lubricant, a humectant, a sweetener, a flavor enhancer, an emulsifier, a suspending agent, a preservative, and a combination thereof.

The pharmaceutical composition, or the antibody or its antigen-binding fragment, may be administered orally or parenterally. Parenteral administration may be carried out via intravenous, subcutaneous, intramuscular, intraperitoneal, intradermal, local, intranasal, intrapulmonary, and intrarectal routes. For oral administration, however, the pharmaceutical composition is preferably coated or formulated to protect the active ingredient from being degraded in the stomach because proteins or peptides are digested by pepsin. In addition, the administration may be performed with the aid of an instrument adapted for delivering the pharmaceutical composition to target cells.

The pharmaceutical composition, or the antibody or its antigen-binding fragment, may be administered in a pharmaceutically effective amount. The term "pharmaceutically effective amount", as used herein, refers to an amount at which the active ingredient can exert a desired effect. The pharmaceutically effective amount may be determined in consideration of various factors comprising the type of formulation, the type of administration, the patient's age, weight, and sex, the severity of the disorder being treated, diet, the time and interval of administration, the route of administration, the rate of excretion, and sensitivity.

The content of the anti-Ang2 antibody or its antigen-binding fragment in the pharmaceutical composition may vary depending on various factors comprising the type of formulation, the type of administration, the patient's age, weight, and sex, the severity of the disorder being treated, diet, the time and interval of administration, the route of administration, the rate of excretion, and sensitivity. For example, the daily dose of the anti-Ang2 antibody or an antigen-binding fragment thereof may be on the order of 0.001 to 1000 mg/kg, particularly on the order of 0.01 to 100 mg/kg, and more particularly on the order of 0.1 to 50 mg/kg, but is not limited thereto. A daily dose may be formulated into a unit dose form or distributed into separate dose forms, or may be comprised within a multiple dose package.

The pharmaceutical composition may be formulated into a solution in an oily or aqueous medium, a suspension, a syrup, an emulsion, an elixir, a powder, a granule, a tablet, or a capsule, and in the context of formulation, a dispersant or a stabilizer may be further employed.

Particularly, the pharmaceutical composition comprising the anti-Ang2 antibody or its antigen-binding fragment can be formulated into immunoliposomes. Liposomes comprising an antibody can be prepared using methods that are well-known in the art. The immunoliposomes may be produced from a lipid composition comprising phosphatidylcholine, cholesterol, and PEGylated phosphatidylethanolamine by reverse-phase evaporation. For example, Fab' can be conjugated to liposomes by disulfide reformation.

### EXAMPLES

Hereafter, the present invention will be described in detail by examples.

The following examples are intended merely to illustrate the invention and are not construed to restrict the invention.

### Example 1. Preparation of a mouse anti-Ang2 antibody, 10D6

### 1.1. Immunization of a mouse

A human Ang2 protein (R&D systems; 623-AN-025/CF) was administered to 5-week-old BALB/c mice along with an adjuvant to induce an immune response and hybridomas that produce an individual anti-Ang2 antibody were prepared according to the methods of Schwaber, et al (Schwaber, J and Cohen, E. P., "Human x Mouse Somatic Cell Hybrid Clones Secreting Immunoglobulins of Both Parental Types," Nature, 244 (1973), 444-447).

More specifically, to obtain immunized mice necessary for developing hybridoma cell lines, 100 µg (microgram) of human Ang2 protein (R&D Systems) mixed with the same amount of a complete Freund's adjuvant was administered via an intraperitoneal injection to each of five 4∼6-week-old BALB/c mice (Japan SLC, Inc.). After two weeks, the antigen (half the previously injected amount) mixed with an incomplete Freund's adjuvant using the same method as described above was administered to each mouse via an intraperitoneal injection. After one additional week, a final boosting was performed and three days later, blood was collected from the tail of each mouse to obtain serum, which was then diluted at 1/1000 with PBS and subjected to an ELISA to verify that the titer of an antibody recognizing Ang2 was increased. From the results, mice in which a sufficient amount of the antibody was obtained were selected, and a cell fusion process was performed on the selected mice.

Three days before the cell fusion experiment, a mixture of 50 µg of PBS and 100 µg of human Ang2 protein (R&D systems) was administered via an intraperitoneal injection to BALB/c mice (Japan SLC, Inc.), and after each immunized mouse was anesthetized, its spleen located on the left side of the body was extracted. The extracted spleen was ground with a mesh to isolate cells, which were mixed with a culture medium (DMEM, Hyclon) to prepare a spleen cell suspension. The suspension was centrifuged to collect a cell layer. The obtained 1×10⁸ spleen cells were mixed with 1×10⁷ myeloma cells (Sp2/0), and the mixture was centrifuged to precipitate the cells. The centrifuged precipitate was slowly dispersed, treated with 1 ml of 45% polyethylene glycol (PEG 1500) contained in a culture medium (DMEM), and maintained at 37°C. for one minute before adding 1 ml of a culture medium (DMEM). Subsequently, 10 ml of the culture medium (DMEM) was added for 1 minute to the resultant, which was incubated in a water bath at 37°C for 5 minutes and then re-centrifuged after the total volume was adjusted to 50 ml. The resulting cell precipitate was re-suspended in an isolation medium (HAT medium) at a concentration of 1∼2×10⁵/ml, and the resultant suspension was distributed at 0.1 ml to the each well of a 96-well plate, which was then incubated in a carbon dioxide incubator at 37°C to prepare the hybridoma cell groups.

### 1.2. Production and purification of a monoclonal antibody

The above obtained individual antibody producing hybridomas were screened using a typical ELISA format and 95 anti-Ang2 monoclonal antibodies among the hybridomas differentiated from their mother hybridomas were selected based on their binding potential with Ang2.

More specifically, to select the hybridoma cells that specifically react only to Ang2 protein among the hybridoma cell groups prepared in Example 1.1 above, an ELISA assay method using a human Ang2 protein as an antigen was used for screening.

Human Ang2 protein was added at 100 ng per each well to a microtiter plate to be adhered to the surface of the plate, and unreacted antigens were removed by washing. 50 microliters of the hybridoma cell culture obtained in Example 1 above was added to each well to react for 1 hour and then, the wells were sufficiently washed with phosphate buffered saline-TWEEN 20 (PBST) solution to remove unreacted culture solution. Goat anti-mouse IgG-horseradish peroxidase (goat anti-mouse IgG-HRP) was added thereto, a reaction was allowed to occur at a room temperature for 1 hour and then, washing was performed with the TBST solution. Subsequently, a substrate solution (OPD) of peroxidase was added to each well to react, and the reaction degree was measured by the absorption at 450 nm using an ELISA reader to repeatedly select hybridoma cell lines that secret antibodies having specifically high binding affinity only to human Ang2 protein. A limiting dilution was performed on the hybridoma cell lines obtained through repetitive selection to obtain final 58 clones of hybridoma cell lines producing monoclonal antibodies.

Each hybridoma obtained above was cultured in DMEM (Dulbeco's Modified Eagle's Medium) and then, the culture solutions were collected and subjected to Protein G-affinity chromatography to purify anti-Ang2 monoclonal antibodies produced from each hybridoma.

First, the hybridoma cells cultured in 50 ml of culture medium (DMEM) containing 10% (v/v) FBS were centrifuged to obtain a cell precipitate, which was washed at least twice with 20 ml of PBS to remove the FBS. The cell precipitate was re-suspended in 50 ml of the culture medium (DMEM) and then incubated in a carbon dioxide incubator at 37°C for 3 days. Subsequently, the cell culture was centrifuged to remove the antibody-producing cells, and the culture medium including the secreted antibodies was isolated and then, stored at 4°C or used directly. Antibodies were purified from 50 to 300 ml of the culture medium using an AKTA purification device (GE Healthcare) equipped with an affinity column (protein G agarose column; Pharmacia, USA). The purified antibodies were stored for subsequent use after replacing the supernatant with PBS using a filter for protein aggregation (Amicon), and used for the following examples.

### 1.3. Examination of functions of monoclonal antibodies and selection of mouse anti-Ang2 antibody 10D6

A test for analyzing an influence of the anti-Ang2 antibody on Tie2 phosphorylation was conducted using a cell-based assay.

HUVEC (ATCC) cells (1X10⁵ cells) were cultured in a 100 mm culture dish using EGM-2 (Lonza) media at 37°C and when they reached 80∼90% confluency, the media were replaced with serum-free medium (Lonza) and cultured at 37°C for 6 to 16 hours. The dish was washed once with PBS and after the replacement with 1 nM sodium orthovanadate (Sigma)-mixed serum free media (Lonza), they were further cultured for 10 min. After the cells were washed once again with PBS, the cultured cells were treated with a mixture prepared by mixing the anti-Ang2 antibody (10D6) having various concentrations (600∼0.06 nM) with 40 nM of Ang2 protein (R&D systems) and letting them stand for 20 min. and further cultured for 10 min.

The cells were washed using PBS, treated with 400 µl of a lysis buffer (Roche), collected to a tube to be dissolved at 4°C for 30 min. and then, centrifuged at 13,000 rpm for 15 min. to measure a supernatant using Nanodrop. 1 µg of Tie2 antibody (R&D system) was added to 0.8 mg of a cell lysate, which was then overnight reacted at 4°C and then subjected to immunoprecipitation by the addition of protein A bead (GE Healthcare) thereto. The obtained reactant was centrifuged at 13,000 rpm for 15 min. to obtain a pellet, which was washed two to three times with a lysis buffer (Roche), added to a sample buffer (Invitrogen) mixed with a reducing agent, and boiled at 95°C for 5 min., and then, applied to NuPAGE Novex 4-12% Bis-Tris gel (Invitrogen) and transferred onto Nitrocellulose membrane (Invitrogen).

To examine the presence of the phosphorylation of Tie2, the membranes were blocked with PBST mixed with 3% (v/v) skim milk (Sigma) for 30 min. and identified using an HRP-conjugated anti-phospho tyrosine antibody (Millipore). For Tie2 identification, the blots were reacted in a stripping buffer (Thermo) for 15 min, then blocked again and identified using an anti-Tie2 antibody (Santa cruz). An antibody, which shows more intensive ability to induce a phosphorylation of Tie2 receptor when it is added together with Ang2 at the concentration of 60nM, compared to the case treated with Ang2 only, was selected and named as 10D6.

The hybridoma producing 10D6 was deposited in the Korean Cell Line Bank located at Yongon-dong, Chongno-gu, Seoul, South Korea, as of April 23, 2013 and received accession number KCLRF-BP-00295.

### 1.4. Analysis of binding affinity of mouse antibody 10D6 to Ang2

The binding affinity of the above antibody to human Ang2 protein was measured by an surface plasmon resonance (SPR) method using a BIAcore T100 (GE Healthcare). The SPR method uses refractive index change of light which passes a sensor chip according to the state of materials coated onto the sensor chip, and if an antigen or an antibody is flowed onto a chip coated with the antigen or antibody, it causes changes in refractive index due to their binding and Kd values are thus calculated from the measured values.

First, anti-His antibody was immobilized on a CM5 sensor chip (GE healthcare) up to 8,000 RU levels using a pH 5.0 acetate solution and an amine coupling kit (GE Healthcare). 6 µg/ml of a recombinant hAng2 (C-His, R&D Systems) protein was flowed onto the chip to be captured at 100 to 200 RU levels. The antibody obtained in Example 2 above was diluted serially to twice each time starting from 100 nM concentration and it was each flowed onto the chip to allow it to be bound to (on), dissociated from (off), and regenerated (using 10 mM NaOH solution) from the antigen captured on the sensor chip, thereby to measure antigen-antibody affinity. With regard to hAng2, such experiments were conducted, and the results are as shown in the following Table 4.

**[Table 4]**

| antibody | hAng2 (Kd) |
|---|---|
| SAIT-ANG2-AB-m10D6 | 8.0 nM |

### Example 2: Gene cloning of mouse antibody 10D6

RNA was obtained using RNeasy mini kit (Qiagen) from the antibody-producing hybridoma (2x10⁶ cells) obtained from Example 1.3 above. Then, by using this as a template, only the gene sequence of the heavy chain and light chain variable regions of the monoclonal antibody to be produced in the hybridoma was amplified using a OneStep RT-PCR kit (Qiagen), a Mouse Ig-Primer Set (Novagen), and a thermocycler (GeneAmp PCR System 9700, Applied Biosystem) under the following conditions: 5 min. at 94°C; [30 min. at 50°C, 15 min. at 95°C], [1 min. at 94°C, 1 min. at 50°C, 2 min. at 72°C] x 35 cycles; 6 min. at 72°C; cooling to 4°C.

The PCR products obtained from each reaction were subjected to a direct DNA sequencing to obtain the amino acid sequences of the CDR, heavy chain variable regions and light chain variable regions of the antibody, and nucleotide sequences encoding them, and the obtained results are set forth in the following Tables 5 to 8.

**[Table 5]**

| Antibody | Amino acid sequence of heavy chain CDR | | |
|---|---|---|---|
| | CDRH1-KABAT | CDRH2-KABAT | CDRH3-KABAT |
| SAIT-ANG2-AB-m10D6 | SDYAWN (SEQ ID NO:1) | YINYSGNTDYNPSLKS (SEQ ID NO: 2) | GNFEGAMDY (SEQ ID NO:3) |

**[Table 6]**

| Antibody | Amino acid sequence of light chain CDR | | |
|---|---|---|---|
| | CDRL1-KABAT | CDRL2-KABAT | CDRL3-KABAT |
| SAIT-ANG2-AB-m10D6 | KASQSVSNDVA (SEQ ID NO:4) | YASNRYP (SEQ ID NO: 5) | QQDYSSPWT (SEQ ID NO: 6) |

**[Table 7]**

| Antibody | Sequence of heavy chain variable region |
|---|---|
| SAIT-ANG2-AB-m10D6 | DVQLQESGPDLVKPSQSLSLTCTVTGYSITSDYAWNWIRQFPGNKL EWMGYINYSGNTDYNPSLKSRSSITRDTSKNQFFLQLNSVTTGDTA TYYCARGNFEGAMDYWGQGTSVTVSS(SEQ ID NO: 7) |
| | GATGTGCAGCTTCAGGAGTCGGGACCTGACCTGGTGAAACCTTC TCAGTCTCTGTCCCTCACCTGCACTGTCACTGGCTACTCAATCAC CAGTGATTATGCCTGGAACTGGATCCGGCAGTTTCCAGGAAACA AACTGGAGTGGATGGGCTACATAAACTACAGTGGTAACACTGAC TACAACCCATCTCTCAAAAGTCGAAGCTCTATCACTCGAGACAC ATCCAAGAACCAGTTCTTCCTGCAGTTGAATTCTGTGACTACTGG GGACACAGCCACATATTACTGTGCAAGAGGTAACTTCGAAGGTG CTATGGACTACTGGGGTCAAGGAACCTCAGTCACCGTCTCCTCA( SEQ ID NO: 8) |

**[Table 8]**

| Antihody | Sequence of light chain variable region |
|---|---|
| SAIT-ANG2-AB-m10D6 | SIVMTQTPKFLLVSAGDRVTITCKASQSVSNDVAWYQQKPGQSPK LLIYYASNRYPGVPDRFTGSGYGTDFTFTISTVQAEDLAVYFCQQD |
| | YSSPWTFGGGTKLEIK(SEQ ID NO: 9) |
| | agtattgtgatgacccagactcccaaattcctgcttgtatcagcaggagacagggttaccataacctgcaag gccagtcagagtgtgagtaatgatgtagcttggtaccaacagaagccagggcagtctcctaaactgctgat atactatgcatccaatcgctaccctggagtccctgatcgcttcactggcagtggatatgggacggatttcactt tcaccatcagcactgtgcaggctgaagacctggcagtttatttctgtcagcaggattatagctctccgtggac gttcggtggaggcaccaagctggaaatcaaa(SEQ ID NO: 10) |

(In above Tables 6 and 7, underlined bold letters are CDR1, CDR2, and CDR3 in sequence)

Based on the sequence information obtained above, single chain DNAs encoding the heavy chain variable region and the light chain variable region, respectively, were prepared, and cloned into vectors comprising a human kappa constant region coding gene and a CH1 region coding gene of human IgG1, respectively. In particular, a DNA fragment having the heavy chain variable region coding nucleotide sequence (SEQ ID NO: 8) was cloned into a vector of pOptiVEC^{™}-TOPO TA Cloning Kit comprised in OptiCHO^{™} Antibody Express Kit (Cat no. 12762-019; Invitrogen), and a DNA fragment having the light chain variable region coding nucleotide sequence (SEQ ID NO: 10) was cloned into a vector of pcDNA^{™}3.3-TOPO TA Cloning Kit(Cat no. 8300-01), using EcoRI(NEB, R0101S) and XhoI(NEB, R0146S), to construct a vector comprising the heavy chain variable region and a vector comprising the light chain variable region for expressing a chimeric antibody.

### Example 3: Preparation of an scfv of mouse antibody 10D6

A gene for producing an scFv fragment using the heavy chain variable region and the light chain variable region of mouse antibody 10D6 was designed. The heavy chain variable region (amino acid sequence: SEQ ID NO: 7; coding nucleotide sequence: SEQ ID NO: 8) and the light chain variable region (amino acid sequence: SEQ ID NO: 9; coding nucleotide sequence: SEQ ID NO: 10) were linked to form 'VH-linker-VL' construct, and the linker is designed to have the amino acid sequence of 'GGGGSGGGGSGGGGS(SEQ ID NO: 76)'. The amino acid sequence of the designed 'VH-linker-VL' (scFv of 10D6) is represented in SEQ ID NO: 80 and the coding nucleotide sequence thereof is represented in SEQ ID NO: 81.

### Example 4: Preparation of gene library for affinity maturation

### 4.1. Selection of target CDR and preparation of primers

To perform affinity maturation, six complementary determining regions (CDRs) were defined from the prepared mouse antibody 10D6 according to the 'Kabat numbering' rule. The CDRs are summarized in Table 9:

**[Table 9]**

| CDR | Amino acid sequence |
|---|---|
| CDR-H1 | SDYAWN(SEQ ID NO: 1) |
| CDR-H2 | YINYSGNTDYNPSLKS(SEQ ID NO: 2) |
| CDR-H3 | GNFEGAMDY(SEQ ID NO: 3) |
| CDR-L1 | KASQSVSNDVA(SEQ ID NO: 4) |
| CDR-L2 | YASNRYP(SEQ ID NO: 5) |
| CDR-L3 | QQDYSSPWT(SEQ ID NO:6) |

For use in the introduction of random sequences into the CDRs of the antibody, primers were designed as follows. Conventionally, N codons were utilized to introduce bases at the same ratio (25% A, 25% G, 25% C, 25% T) into desired sites of mutation. In this experiment, the introduction of random bases into the CDRs of 10D6 was conducted in such a manner that, of the three nucleotides per codon in the wild-type polynucleotide encoding each CDR, the first and second nucleotides conserved over 85% of the entire sequence while the other three nucleotides were introduced at the same percentage (each 5%) and that the same possibility was imparted to the third nucleotide (33 % G, 33 % C, 33 % T).

### 4.2. Construction of gene library of scfv of 10D6 antibody

The construction of antibody gene libraries through the introduction of random sequences was carried out using the primers synthesized in the same manner as in Example 4.1. Two PCR products were obtained using a polynucleotide covering the 10D6 scFv (SEQ ID NO: 81) as a template (see following figure), and were subjected to overlap extension PCR to give scFv library genes for 10D6 antibodies in which only desired CDRs were mutated.

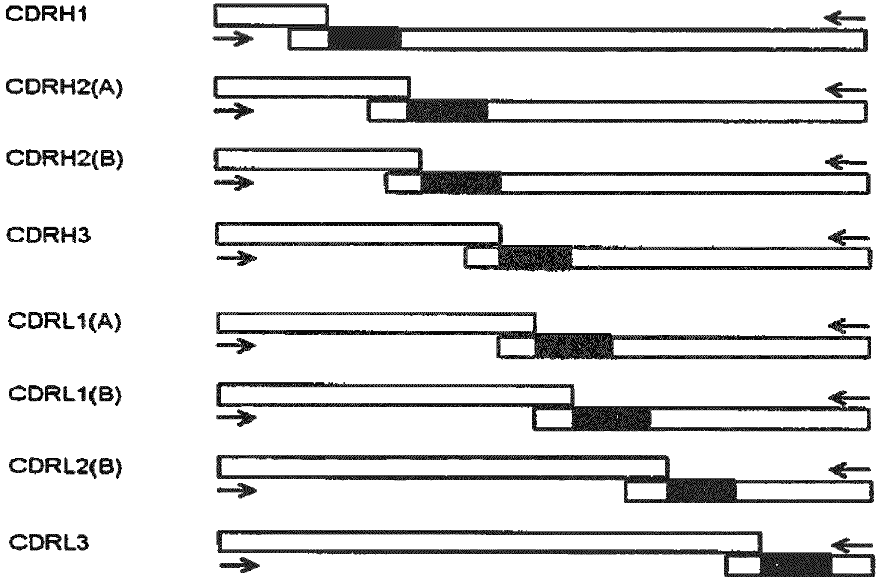
CDRH1
CDRH2(A)
CDRH2(B)
CDRH3
CDRL1(A)
CDRL1(B)
CDRL2(B)
CDRL3

10⁷∼10⁸ libraries targeting each of the six CDRs prepared from the scFv library genes were constructed.

The affinity for Ang2 of each library was compared to that of the wild-type. Most libraries were lower in affinity for Ang2, compared to the wild-type. However, in some mutants, the affinity for Ang2 was retained.

### Example 5: Selection of antibody with improved affinity from libraries

Among the scFv libraries provided in Example 4, the scFv fragments showing upper 1.0 percent of affinity to Ang-2 were selected, and this process was repeated four times. The nucleotide sequence of each of the selected scFv was analyzed. The obtained nucleotide sequences are summarized in Table 10, and were converted into IgG forms (a heavy chain constant region: constant region of human IgG1, a light chain constant region: constant region of human KAPPA Chain). Five antibodies which were respectively produced from clones VH-6.6, VH-6.7, VL-(6.11), VL-(6.17), and VL-HU1(6.22) were used in the subsequent experiments.

**[Table 10]**

| clones | Library constructed | CDR sequence |
|---|---|---|
| VH-6.6 | CDR-H2 | KISYSGKTDYNPSLKS(SEQ ID NO:14) |
| VH-6.7 | CDR-H2 | KINYAGNTDYNPSLKS(SEQ ID NO: 15) |
| VL-(6.11) | CDR-L1 | KASQSVSNDVH(SEQ ID NO: 16) |
| VL-(6.17) | CDR-L3 | QHDYSSPFT(SEQ ID NO: 19) |
| VL-(6.22) | CDR-L1+ CDR-L3 | KASQSVSNDVH(SEQ ID NO: 16)+ QHDYSSPFT(SEQ ID NO: 19) |

### Example 6: Preparation of humanized antibody 10D6-HU1, 10D6-HU2, 10D6-HU3, and 10D6-HU5, from mouse antibody 10D6

### 6.1. Heavy chain humanization

To design three domains 10D6-HU1 Heavy, 10D6-HU2-heavy, and 10D6-HU5-heavy, human germline genes which share the highest identity/homology with the VH gene of the mouse antibody 10D6 purified were analyzed through an Ig BLAST (IgBLAST online database tool, maintained by National Center for Biotechnology Information (NCBI), Bethesda, MD). The analysis results revealed that IGHV4-b*01 (DP-67; accession number: Z12367) has an identity/ identity/homology of 72% at the amino acid level. CDR-H1(SEQ ID NO: 1), CDR-H2(SEQ ID NO: 2), and CDR-H3(SEQ ID NO: 3) of the mouse antibody 10D6 were defined according to Kabat numbering.

A design was made to introduce the CDR of the mouse antibody 10D6 into the framework of IGHV4-b*01 (named as 10D6-HU1; SEQ ID NO: 77;
QVQLQESGPGLVKPSETLSLTCAVSGYSISSDYAWNWIRQPPGKGLEWIGYIN
YSGNTDYNPSLKSRVTISVDTSKNQFSLKLSSVTAADTAVYYCARGNFEGAM
DYWGQGTLVTVSS). Hereupon, a back mutation to the amino acid sequence of the mouse 10D6 were conducted at positions 30 (S→T). to establish antibody 10D6-HU5 (SEQ ID NO: 56). Then, 10D6-HU5 was further mutated at positions 48 (I→M), 67 (V→S), and 71 (V→R), to establish 10D6-HU2(SEQ ID NO: 78;
QVQLQESGPGLVKPSETLSLTCAVSGYSITSDYAWNWIRQPPGKGLEWMGYI
NYSGNTDYNPSLKSRSTISRDTSKNQFSLKLSSVTAADTAVYYCARGNFEGA
MDYWGQGTLVTVSS).

For use in designing 10D6-HU3-heavy, human antibody frameworks were analyzed by a BLAST search. The result revealed that the Herceptin backbone, which known to show very low immunogenicity of about 0.1% level among the pre-existing humanized antibodies, is very similar in framework and sequence to the mouse antibody 10D6. CDR-H1, CDR-H2, and CDR-H3 of the mouse antibody 10D6 were defined according to Kabat numbering and introduced into the Herceptin backbone to construct H4-heavy (SEQ ID NO: 42), wherein back mutations were conducted at positions 27 (F-Y), 28(N→S), 30(K→T), 48(V→M), 49(A→G), 67 (F→S), 71(A→R), 78(A→F), and 93(S→A), to establish 10D6-HU3(SEQ ID NO: 79;
EVQLVESGGGLVQPGGSLRLSCAASGYSITSDYAWNWVRQAPGKGLEWMG
YINYSGNTDYNPSLKSRSTISRDTSKNTFYLQMNSLRAEDTAVYYCARGNFEG
AMDYWGQGTLVTVSS).

### 6.2. Light chain humanization

To design a H1-light, human germline genes which share the highest identity/homology with the VL gene of the mouse antibody 10D6 were analyzed through an Ig BLAST. The analysis results revealed IGKV1-39*01(O12; accession number: X59315) has an identity/ identity/homology of 66% at the amino acid level. CDR-L1(SEQ ID NO: 4), CDR-L2(SEQ ID NO: 5), and CDR-L3(SEQ ID NO: 6) of the mouse antibody 10D6 were defined according to Kabat numbering. A design was made to introduce the CDR of the mouse antibody 10D6 into the framework of IGKV1-39*01.

Thereafter, DNA fragments of heavy chains (10D6-VHHU1, 10D6-VHHU2, 10D6-VHHU3, and 10D6-VHHU5) were respectively cloned into a vector of pOptiVEC^{™}-TOPO TA Cloning Kit enclosed in an OptiCHO^{™} Antibody Express Kit (Cat no. 12762-019, Invitrogen) using EcoRI(NEB, R0101S) and nheI(NEB, R0131), and a DNA fragment of a light chain (10D6-VLHU1(SEQ ID NO: 57), coding sequence: SEQ ID NO: 69) was cloned into a vector of pcDNATM3.3-TOPO TA Cloning Kit using EcoRI(NEB, R0101S) and XhoI(NEB, R0146S), to construct recombinant vectors for expressing a humanized antibody.

The constructed vectors were amplified using a Qiagen Maxiprep kit (Cat No. 12662), and the vectors including the heavy chain and the vector including the light chain were added to 293T cells (2.5 x 10⁷) at a ratio of about 4:1 (about 80 ug:20 ug) with 360 ul of 2 M CaCl₂ and were transfected. Next, the mixture was cultured in a DMEM medium with 10% (w/v) FBS at 37°C in 5% (v/v) CO₂ conditions for 5 hours, and then cultured in a DMEM medium without FBS at 37°C in 5% (v/v) CO₂ conditions for 48 hours.

The cultured cells were centrifuged, and 100 ml of each supernatant was purified using AKTA Prime (GE healthcare). Protein A column (GE healthcare, 17-0405-03) was placed in the AKTA Prime, and the cultured solution was flowed at a flow rate of 5 ml/min and was eluted with IgG elution buffer (Thermo Scientific, 21004). The buffer was replaced with a PBS buffer, and thus final humanized antibodies 10D6-HU1, 10D6-HU2, 10D6-HU3, and 10D6-HU5 were purified.

### Example 7: Incorporation of the selected CDRs into humanized antibody and transformation to IgG

The selected CDRs were incorporated into the heavy chain and the light chain of the humanized antibodies. Polynucleotides encoding the heavy chain of the antibodies were synthesized by Bioneer, Inc. so as to consist of 'EcoRI-signal sequence-VH-NheI-CH-XhoI' (SEQ ID NOs: 64-68). Polynucleotides encoding the light chain of the antibodies were synthesized by Bioneer, Inc. so as to consist of 'EcoRI-signal sequence-VL-BsiWI-CL-XhoI' (SEQ ID NOs: 69-71). The polynucleotides (SEQ ID NOs: 64-68) encoding the heavy chain were respectively cloned into a vector of pOptiVEC^{™}-TOPO TA Cloning Kit included in OptiCHO^{™} Antibody Express Kit (Cat no. 12762-019; Invitrogen), and the polynucleotides (SEQ ID NOs: 69-71) encoding the light chain were respectively cloned into a vector of pcDNA^{™}3.3-TOPOTA Cloning Kit(Cat no. 8300-01), using EcoRI(NEB, R0101S) and XhoI(NEB, R0146S), to establish vectors for expressing affinity matured antibodies.

The constructed vectors were amplified using a Qiagen Maxiprep kit (Cat No. 12662), and the vectors including the heavy chain and the vector including the light chain were added to 293T cells (2.5 x 10⁷) at a ratio of about 4:1 (about 80 ug:20 ug) with 360 ul of 2 M CaCl₂ and were transfected. Next, the mixture was cultured in a DMEM medium with 10% (w/v) FBS at 37°C in 5% (v/v) CO₂ conditions for 5 hours, and then cultured in a DMEM medium without FBS at 37°C in 5% (v/v) CO₂ conditions for 48 hours.

The cultured cells were centrifuged, and 100 ml of each supernatant was purified using AKTA Prime (GE healthcare). Protein A column (GE healthcare, 17-0405-03) was placed in the AKTA Prime, and the cultured solution was flowed at a flow rate of 5 ml/min and was eluted with IgG elution buffer (Thermo Scientific, 21004). The buffer was replaced with a PBS buffer, and thus final affinity-matured antibodies h10D6-Opti-1, h10D6-Opti-2, h10D6-Opti-3, and h10D6-Opti-4 were purified.

**[Table 11]**

| Clone | Antibody sequence (VH) | Antibody sequence (VL) |
|---|---|---|
| h10D6-OPTI-1 | >HU2-6.6 | >HU1 |
| | QVQLQESGPGLVKPSETLSLTCAVSGYS ITSDYAWNWIRQPPGKGLEWMGKISYS GKTDYNPSLKSRSTISRDTSKNQFSLKL SSVTAADTAVYYCARGNFEGAMDYW GQGTLVTVSS(SEQ ID NO: 52) | DIQMTQSPSSLSASVGDRVTITCKASQSV SNDVAWYQQKPGKAPKLLIYYASNRYP GVPSRFSGSGSGTDFTLTISSLQPEDFATY YCQQDYSSPWTFGQGTKLEIK(SEQ ID NO: 57) |
| | (coding nucleotide sequence) | (coding nucleotide sequence) |
| | CAGGTGCAACTGCAGGAGTCAGGCCC CGGCCTGGTAAAACCTTCTGAAACGCT CTCACTTACCTGTGCCGTTAGTGGATA CTCTATCACTTCCGACTACGCTTGGAA TTGGATTCGGCAGCCTCCAGGCAAAG GGCTGGAATGGATGGGAAAGATTTCC TATTCCGGTAAGACTGACTACAATCCC AGTCTGAAGAGCAGGTCAACAATCTC CAGAGACACCAGCAAGAATCAGTTTT CCCTGAAATTGTCCTCGGTGACAGCAG CGGATACCGCAGTGTATTATTGCGCCC GCGGTAACTTCGAGGGAGCTATGGAT TACTGGGGGCAGGGTACTCTCGTCACT GTGAGCAGC(SEQ ID NO: 64) | GACATCCAGATGACCCAGTCTCCATCC TCCCTGTCTGCATCTGTAGGAGACAGA GTCACCATCACTTGCAAGGCCAGTCAG AGTGTGAGTAATGATGTAGCTTGGTAT CAGCAGAAACCAGGGAAAGCCCCTAA GCTCCTGATCTATTATGCATCCAATCGC TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 69) |
| h10D6-OPTI-2 | >HU2-6.7 | >HU1 |
| | QVQLQESGPGLVKPSETLSLTCAVSGYS ITSDYAWNWIRQPPGKGLEWMGKINY AGNTDYNPSLKSRSTISRDTSKNQFSLK LSSVTAADTAVYYCARGNFEGAMDYW GQGTLVTVSS(SEQ ID NO: 53) | DIQMTQSPSSLSASVGDRVTITCKASQSV SNDVAWYQQKPGKAPKLLIYYASNRYP GVPSRFSGSGSGTDFTLTISSLQPEDFATY YCQQDYSSPWTFGQGTKLEIK(SEQ ID NO: 57) |
| | (coding nucleotide sequence) | (coding nucleotide sequence) |
| | [?] CAGGTGCAACTGCAGGAGTCAGGCCC CGGCCTGGTAAAACCTTCTGAAACGCT CTCACTTACCTGTGCCGTTAGTGGATA CTCTATCACTTCCGACTACGCTTGGAA TTGGATTCGGCAGCCTCCAGGCAAAG GGCTGGAATGG ATGGGAAAGATTAACTATGCCGGTAA CACTGACTACAATCCCAGTCTGAAGA GCAGGTCAACAATCTCCAGAGACACC AGCAAGAATCAGTTTTCCCTGAAATTG TCCTCGGTGACAGCAGCGGATACCGC AGTGTATTATTGCGCCCGCGGTAACTT CGAGGGAGCTATGGATTACTGG GGGCAGGGTACTCTCGTCACTGTGAG CAGC(SEQ ID NO: 65) | GACATCCAGATGACCCAGTCTCCATCC TCCCTGTCTGCATCTGTAGGAGACAGA GTCACCATCACTTGCAAGGCCAGTCAG AGTGTGAGTAATGATGTAGCTTGGTAT CAGCAGAAACCAGGGAAAGCCCCTAA GCTCCTGATCTATTATGCATCCAATCGC TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 69) |
| h10D6-OPTI-43 | >HU2 -6.6 | >HU1-6.11 |
| | QVQLQESGPGLVKPSETLSLTCAVSGYS ITSDYAWNWIRQPPGKGLEWMGKISYS | DIQMTQSPSSLSASVGDRVTITCKASQSV SNDVHWYQQKPGKAPKLLIYYASNRYP |
| | GKTDYNPSLKSRSTISRDTSKNQFSLKL SSVTAADTAVYYCARGNFEGAMDYW GQGTLVTVSS(SEQ ID NO: 52) | GVPSRFSGSGSGTDFTLTISSLQPEDFATY YCQQDYSSPWTFGQGTKLEIK(SEQ ID NO: 58) |
| | (coding nucleotide sequence) | (coding nucleotide sequence) |
| | CAGGTGCAACTGCAGGAGTCAGGCCC CGGCCTGGTAAAACCTTCTGAAACGCT CTCACTTACCTGTGCCGTTAGTGGATA CTCTATCACTTCCGACTACGCTTGGAA TTGGATTCGGCAGCCTCCAGGCAAAG GGCTGGAATGG ATGGGAAAGATTTCCTATTCCGGTAAG ACTGACTACAATCCCAGTCTGAAGAG CAGGTCAACAATCTCCAGAGACACCA GCAAGAATCAGTTTTCCCTGAAATTGT CCTCGGTGACAGCAGCGGATACCGCA GTGTATTATTGCGCCCGCGGTAACTTC GAGGGAGCTATGGATTACTGG GGGCAGGGTACTCTCGTCACTGTGAG CAGC(SEQ ID NO: 64) | GACATCCAGATGACCCAGTCTCCATCC TCCCTGTCTGCATCTGTAGGAGACAGA GTCACCATCACTTGCAAGGCCAGTCAG AGTGTGAGTAATGATGTACATTGGTAT CAGCAGAAACCAGGGAAAGCCCCTAA GCTCCTGATCTATTATGCATCCAATCGC TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 70) |
| h10D6-OPTI-55 | >HU2 -6.7 | HU1-6.11 |
| | QVQLQESGPGLVKPSETLSLTCAVSGYS ITSDYAWNWIRQPPGKGLEWMGKINY AGNTDYNPSLKSRSTISRDTSKNQFSLK LSSVTAADTA VYYCARGNFEGAMDYW GQGTLVTVSS(SEQ ID NO: 53) | DIQMTQSPSSLSASVGDRVTITCKASQSV SNDVHWYQQKPGKAPKLLIYYASNRYP GVPSRFSGSGSGTDFTLTISSLQPEDFATY YCQQDYSSPWTFGQGTKLEIK(SEQ ID NO: 58) |
| | (coding nucleotide sequence) | (coding nucleotide sequence) |
| | CAGGTGCAACTGCAGGAGTCAGGCCC CGGCCTGGTAAAACCTTCTGAAACGCT CTCACTTACCTGTGCCGTTAGTGGATA CTCTATCACTTCCGACTACGCTTGGAA TTGGATTCGGCAGCCTCCAGGCAAAG GGCTGGAATGG ATGGGAAAGATTAACTATGCCGGTAA CACTGACTACAATCCCAGTCTGAAGA GCAGGTCAACAATCTCCAGAGACACC AGCAAGAATCAGTTTTCCCTGAAATTG TCCTCGGTGACAGCAGCGGATACCGC AGTGTATTATTGCGCCCGCGGTAACTT CGAGGGAGCTATGGATTACTGG GGGCAGGGTACTCTCGTCACTGTGAG CAGC(SEQ ID NO: 65) | GACATCCAGATGACCCAGTCTCCATCC TCCCTGTCTGCATCTGTAGGAGACAGA GTCACCATCACTTGCAAGGCCAGTCAG AGTGTGAGTAATGATGTACATTGGTAT CAGCAGAAACCAGGGAAAGCCCCTAA GCTCCTGATCTATTATGCATCCAATCGC TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 70) |
| h10D6-OPTI-3 | >HU3-6.6 | >HU1 |
| | EVQLVESGGGLVQPGGSLRLSCAASGY SITSDYAWNWVRQAPGKGLEWMGKIS YSGKTDYNPSLKSRSTISRDTSKNTFYL QMNSLRAEDTAVYYCARGNFEGAMD YWGQGTLVTVSS(SEQ ID NO: 54) | DIQMTQSPSSLSASVGDRVTITCKASQSV SNDVAWYQQKPGKAPKLLIYYASNRYP GVPSRFSGSGSGTDFTLTISSLQPEDFATY YCQQDYSSPWTFGQGTKLEIK(SEQ ID NO: 57) |
| | (coding nucleotide sequence) | (coding nucleotide sequence) |
| | GAGGTTCAGCTGGTCGAAAGCGGTGG GGGACTCGTGCAGCCAGGCGGTTCTCT TAGATTATCATGTGCCGCATCCGGGTA CTCCATCACCTCTGATTATGCATGGAA CTGGGTCAGACAAGCCCCCGGAAAGG GCCTGGAGTGGATGGGGAAGATCTCC TATTCAGGGAAGACAGATTATAATCCT TCGCTGAAAAGCAGATCAACAATTAG | GACATCCAGATGACCCAGTCTCCATCC TCCCTGTCTGCATCTGTAGGAGACAGA GTCACCATCACTTGCAAGGCCAGTCAG AGTGTGAGTAATGATGTAGCTTGGTAT CAGCAGAAACCAGGGAAAGCCCCTAA GCTCCTGATCTATTATGCATCCAATCGC TACCCTGGGGTCCCATCAAGGTTCAGT GGCAGTGGATCTGGGACAGATTTCACT |
| | TAGAGACACTTCTAAAAATACTTTTTA CCTCCAGATGAACAGTCTGCGCGCCG AAGACACCGCCGTGTACTACTGCGCT AGGGGAAATTTCGAGGGAGCTATGGA CTATTGGGGCCAGGGCACGTTGGTAA CCGTGAGCAGC(SEQ ID NO: 66) | CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 69) |
| h10D6-OPTI-4 | >HU3-6.7 | >HU1 |
| | EVQLVESGGGLVQPGGSLRLSCAASGY SITSDYAWNWVRQAPGKGLEWMGKIN YAGNTDYNPSLKSRSTISRDTSKNTFYL QMNSLRAEDTAVYYCARGNFEGAMD YWGQGTLVTVSS(SEQ ID NO: 55) | DIQMTQSPSSLSASVGDRVTITCKASQSV SNDVAWYQQKPGKAPKLLIYYASNRYP GVPSRFSGSGSGTDFTLTISSLQPEDFATY YCQQDYSSPWTFGQGTKLEIK(SEQ ID NO: 57) |
| | (coding nucleotide sequence) | (coding nucleotide sequence) |
| | GAGGTTCAACTGGTAGAGTCCGGGGG CGGCCTGGTCCAGCCAGGAGGAAGCC TGCGGCTCTCTTGTGCCGCCAGCGGGT ATAGTATCACTTCAGATTATGCCTGGA ATTGGGTCCGCCAGGCCCCCGGGAAG GGCTTAGAGTGGATGGGTAAAATTAA TTACGCAGGCAACACCGACTATAATC CTTCACTGAAATCTAGATCCACCATCT CTAGAGATACAAGTAAGAACACCTTT TACTTGCAGATGAATAGCCTCAGGGCT GAAGACACTGCTGTGTACTACTGCGC AAGAGGAAACTTCGAAGGAGCGATGG ATTATTGGGGCCAGGGTACGCTTGTGA CAGTGTCCTCT(SEQ ID NO: 67) | GACATCCAGATGACCCAGTCTCCATCC TCCCTGTCTGCATCTGTAGGAGACAGA GTCACCATCACTTGCAAGGCCAGTCAG AGTGTGAGTAATGATGTAGCTTGGTAT CAGCAGAAACCAGGGAAAGCCCCTAA GCTCCTGATCTATTATGCATCCAATCGC TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 69) |
| h10D6-OPTI-16 | >HU3-6.6 | >HU1-6.11 |
| | EVQLVESGGGLVQPGGSLRLSCAASGY SITSDYAWNWVRQAPGKGLEWMGKIS YSGKTDYNPSLKSRSTISRDTSKNTFYL QMNSLRAEDTAVYYCARGNFEGAMD YWGQGTLVTVSS(SEQ ID NO: 54) | DIQMTQSPSSLSASVGDRVTITCKASQSV SNDVHWYQQKPGKAPKLLIYYASNRYP GVPSRFSGSGSGTDFTLTISSLQPEDFATY YCQQDYSSPWTFGQGTKLEIK(SEQ ID NO: 58) |
| | (coding nucleotide sequence) | (coding nucleotide sequence) |
| | GAGGTTCAGCTGGTCGAAAGCGGTGG GGGACTCGTGCAGCCAGGCGGTTCTCT TAGATTATCATGTGCCGCATCCGGGTA CTCCATCACCTCTGATTATGCATGGAA CTGGGTCAGACAAGCCCCCGGAAAGG GCCTGGAGTGGATGGGGAAGATCTCC TATTCAGGGAAGACAGATTATAATCCT TCGCTGAAAAGCAGATCAACAATTAG TAGAGACACTTCTAAAAATACTTTTTA CCTCCAGATGAACAGTCTGCGCGCCG AAGACACCGCCGTGTACTACTGCGCT AGGGGAAATTTCGAGGGAGCTATGGA CTATTGGGGCCAGGGCACGTTGGTAA CCGTGAGCAGC(SEQ ID NO: 66) | GACATCCAGATGACCCAGTCTCCATCC TCCCTGTCTGCATCTGTAGGAGACAGA GTCACCATCACTTGCAAGGCCAGTCAG AGTGTGAGTAATGATGTACATTGGTAT CAGCAGAAACCAGGGAAAGCCCCTAA GCTCCTGATCTATTATGCATCCAATCGC TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 70) |
| h10D6-OPTI-17 | >HU3-6.7 | >HU1-6.11 |
| | EVQLVESGGGLVQPGGSLRLSCAASGY SITSDYAWNWVRQAPGKGLEWMGKIN YAGNTDYNPSLKSRSTISRDTSKNTFYL QMNSLRAEDTAVYYCARGNFEGAMD YWGQGTLVTVSS(SEQ ID NO: 55) | DIQMTQSPSSLSASVGDRVTITCKASQSV SNDVHWYQQKPGKAPKLLIYYASNRYP GVPSRFSGSGSGTDFTLTISSLQPEDFATY YCQQDYSSPWTFGQGTKLEIK(SEQ ID NO: 58) |
| | (coding nucleotide sequence) | (coding nucleotide sequence) |
| | [?] GAGGTTCAACTGGTAGAGTCCGGGG | GACATCCAGATGACCCAGTCTCCATCC |
| | CGGCCTGGTCCAGCCAGGAGGAAGCC TGCGGCTCTCTTGTGCCGCCAGCGGGT ATAGTATCACTTCAGATTATGCCTGGA ATTGGGTCCGCCAGGCCCCCGGGAAG GGCTTAGAGTGGATGGGTAAAATTAA TTACGCAGGCAACACCGACTATAATC CTTCACTGAAATCTAGATCCACCATCT CTAGAGATACAAGTAAGAACACCTTT TACTTGCAGATGAATAGCCTCAGGGCT GAAGACACTGCTGTGTACTACTGCGC AAGAGGAAACTTCGAAGGAGCGATGG ATTATTGGGGCCAGGGTACGCTTGTGA CAGTGTCCTCT(SEQ ID NO: 67) | TCCCTGTCTGCATCTGTAGGAGACAGA GTCACCATCACTTGCAAGGCCAGTCAG AGTGTGAGTAATGATGTACATTGGTAT CAGCAGAAACCAGGGAAAGCCCCTAA GCTCCTGATCTATTATGCATCCAATCGC TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 70) |
| h10D6-OPTI-42 | >HU5 | >HU1-22 |
| | QVQLQESGPGLVKPSETLSLTCAVSGYS ITSDYAWNWIRQPPGKGLEWIGYINYS GNTDYNPSLKSRVTISVDTSKNQFSLKL SSVTAADTAVYYCARGNFEGAMDYW GQGTLVTVSS(SEQ ID NO: 56) | DIQMTQSPSSLSASVGDRVTITCKASQSV SNDVHWYQQKPGKAPKLLIYYASNRYP GVPSRFSGSGSGTDFTLTISSLQPEDFATY YCQHDYSSPFTFGQGTKLEIK(SEQ ID NO: 59) |
| | (coding nucleotide sequence) | (coding nucleotide sequence) |
| | CAGGTGCAGCTGCAGGAGTCGGGCCC AGGACTGGTGAAGCCTTCGGAGACCC TGTCCCTCACCTGCGCTGTCTCTGGTT ACTCCATCACCAGTGATTATGCCTGGA ACTGGATCCGGCAGCCCCCAGGGAAG GGGCTGGAGTGGATTGGGTACATAAA CTACAGTGGTAACACTGACTACAACC CATCTCTCAAAAGTCGAGTCACCATAT CAGTAGACACGTCCAAGAACCAGTTC TCCCTGAAGCTGAGCTCTGTGACCGCC GCAGACACGGCCGTGTATTACTGTGC GAGAGGTAACTTCGAAGGTGCTATGG ACTACTGGGGTCAAGGAACGCTTGTG ACAGTGTCCTCT(SEQ ID NO: 68) | [?] GACATCCAGATGACCCAGTCTCCATCC TCCCTGTCTGCATCTGTAGGAGACAGA GTCACCATCACTTGCAAGGCCAGTCAG AGTGTGAGTAATGATGTACATTGGTAT CAGCAGAAACCAGGGAAAGCCCCTAA GCTCCTGATCTATTATGCATCCAATCGC TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAT GATTATAGCTCTCCGTTCACGTTCGGTG GAGGCACCAAGGTGGAAATCAAA(SEQ ID NO: 71) |

(In Table 11, the bold letters are CDR1, CDR2, and CDR3 in sequence)

### Example 8: Analysis of binding affinity of selected antibodies

The binding affinity (KD values) of the antibodies to human Ang2 protein was measured by an SPR method using a BIAcore T100 (GE Healthcare). 25 µg/ml anti-His antibody was immobilized on a CM5 sensor chip (GE healthcare) using a pH 5.0 acetate solution and an amine coupling kit (GE Healthcare). 6 µg/ml of a recombinant hAng2 (C-His, R&D Systems) protein was flowed onto the chip to be captured at 100 to 200 RU levels. The antibodies obtained in the above examples were diluted serially to twice each time starting from 100 nM concentration and it was each flowed onto the chip to allow it to be bound to (on), dissociated from (off), and regenerated (using 10 mM NaOH solution) from the antigen captured on the sensor chip, thereby to measure antigen-antibody affinity. The KD values were calculated from the values of kₒₙ k_{off}, and the results are as shown in the following Table 12.

**[Table 12]**

| Antibody | kon (1/Ms) | koff (1/s) | KD (nM) |
|---|---|---|---|
| m10D6 | 2.410x10⁴ | 1.932x10⁻⁴ | 8 |
| 10D6-HU1 | 3.082x10⁴ | 0.002599 | 84 |
| 10D6-HU2 | 7.298x10⁴ | 0.003464 | 47 |
| 10D6-HU3 | 4.503x10⁴ | 0.001938 | 43 |
| 10D6-HU5 | 4.856x10⁴ | 0.003115 | 64 |
| h10D6-OPTI-1 | 4.737x10⁵ | 3.209x10⁻⁴ | 0.68 |
| h10D6-OPTI-2 | 4.237x10⁵ | 1.488x10⁻⁴ | 0.34 |
| h10D6-OPTI-43 | 1.531x10⁶ | 5.760x10⁻⁴ | 0.38 |
| h10D6-OPTI-55 | 6.210x10⁵ | 8.489x10⁻⁵ | 0.14 |
| h10D6-OPTI-3 | 6.239x10⁵ | 3.070x10⁻⁴ | 0.49 |
| h10D6-OPTI-4 | 7.357x10⁵ | 2.460x10⁻⁴ | 0.33 |
| h10D6-OPTI-16 | 4.794x10⁵ | 4.434x10⁴ | 0.92 |
| h10D6-OPTI-17 | 4.600x10⁵ | 3.503x10⁻⁴ | 0.76 |
| h10D6-OPTI-42 | 3.358x10⁵ | 2.862x10⁴ | 0.85 |

As shown in Table 12, the affinity to Ang2 of the mouse antibody 10D6 is about 8 nM, the affinities to Ang2 of the 5 affinity-matured and humanized antibodies are from about 0.14 nM to about 0.92nM. The results indicate that the affinity to Ang2 can be improved at least about 5 times up to about 37 times in the affinity-matured antibodies in an IgG form transformed from a scFv form.

### Example 9: Synthesis of a polynucleotide for preparing an scfv of humanized antibody of 10D6 (Opti-1)

The gene for preparing scFv of a humanized 10D6 antibody was designed using the heavy chain variable region and the light chain variable region of humanized 10D6 antibody Opti-1. The heavy chain variable region (amino acid sequence: Hu2 6.6(SEQ ID NO: 52); coding nucleotide sequence: SEQ ID NO: 64), and the light chain variable region(amino acid sequence: SEQ ID NO: Hu1(SEQ ID NO: 57); coding nucleotide sequence: SEQ ID NO: SEQ ID NO: 69) were linked to form a 'VH-linker-VL' construct, and the linker was designed so as to have the amino acid sequence of 'GGGGSGGGGSGGGGS(SEQ ID NO: 76)'. The polynucleotide (SEQ ID NO: 83) encoding the designed scFv ('VH-linker-VL'; SEQ ID NO: 82) of antibody 10D6 opti-1 was synthesized by Bioneer, Inc.

### Example 10: Preparation of gene library for the secondary affinity maturation

### 10.1. Selection of target CDR and preparation of primers

To perform affinity maturation of antibody 10D6 opti-1, three complementary determining regions (CDRs) were defined from the prepared antibody 10D6 opti-1 according to the 'Kabat numbering' rule. The CDRs are summarized in Table 13:

**[Table 13]**

| CDR | amino acid sequence |
|---|---|
| CDR-L1 | KASQSVSNDVA(SEQ ID NO: 4) |
| CDR-L2 | YASNRYP(SEQ ID NO: 5) |
| CDR-L3 | QQDYSSPWT(SEQ ID NO:6) |

For use in the introduction of random sequences into the CDRs of the antibody, primers were designed as follows. Conventionally, N codons were utilized to introduce bases at the same ratio (25% A, 25% G, 25% C, 25% T) into desired sites of mutation. In this experiment, the introduction of random bases into the CDRs of 10D6 was conducted in such a manner that, of the three nucleotides per codon in the wild-type polynucleotide encoding each CDR, the first and second nucleotides conserved over 85% of the entire sequence while the other three nucleotides were introduced at the same percentage (each 5%) and that the same possibility was imparted to the third nucleotide (33 % G, 33 % C, 33 % T).

### 10.2. Construction of gene library of scfv of 10D6 opti-1 antibody

The construction of antibody gene libraries through the introduction of random sequences was carried out using the primers synthesized in the same manner as in Example 11.1. Two PCR products were obtained using a polynucleotide covering the 10D6 opti-1 scFv(SEQ ID NO: 83) as a template (see following figure), and were subjected to overlap extension PCR to give scFv library genes for 10D6 antibodies in which only desired CDRs were mutated.

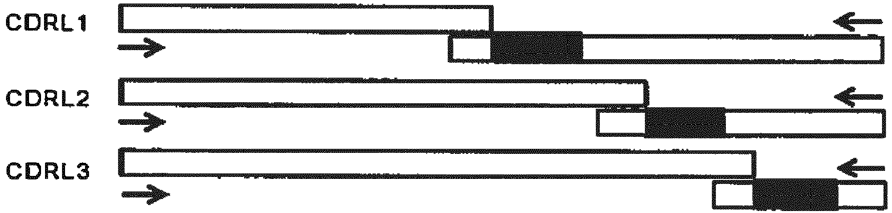
CDRL1
CDRL2
CDRL3

10⁷∼10⁸ libraries targeting each of the six CDRs prepared from the scFv library genes were constructed.

The affinity for Ang2 of each library was compared to that of the wild-type. Most libraries were lower in affinity for Ang2, compared to the wild-type. However, in some mutants, the affinity for Ang2 was retained.

### Example 11: Selection of antibody with improved affinity from libraries

Among the scFv libraries provided in Example 11, the scFv fragments showing upper 1.0 percent of affinity to Ang-2 were selected, and this process was repeated four times. The nucleotide sequence of each of the selected scFv was analyzed. The obtained nucleotide sequences are summarized in Table 14, and were converted into IgG forms (a heavy chain constant region: constant region of human IgG1, a light chain constant region: constant region of human KAPPA Chain). Four antibodies which were respectively produced from clones 10D6_VL-Hu1-2.1, 10D6_VL-Hu1-2.4, 10D6_VL-Hu1-2.7, 10D6_VL-Hu1-2.8 were used in the subsequent experiments.

**[Table 14]**

| Clones | Library constructed | CDR sequence |
|---|---|---|
| 10D6_VL-Hu1-2.1 | CDR-L1 | KASQFVSTDVH(SEQ ID NO: 17) |
| 10D6_VL-Hu1-2.4 | CDR-L2 | YASIPYP(SEQ ID NO: 18) |
| 10D6_VL-Hu1.2.7 | CDR-L1+L2 | KASQSVSNDVH(SEQ ID NO: 16)+YASIPYP(SEQ ID NO: 18) |
| 10D6_VL-Hu1-2.8 | CDR-L1+L2 | KASQFVSTDVH(SEQ ID NO: XX)+YASIPYP(SEQ ID NO: 18) |

### Example 12: Incorporation of the selected CDRs into humanized antibody and transformation to IgG

The selected CDRs were incorporated into the heavy chain and the light chain of the humanized antibodies. The heavy chain was derived from the antibody cloned with Hu2-6.6 or Hu3-6.6. Polynucleotides encoding the light chain of the antibodies were synthesized by Bioneer, Inc. so as to consist of 'EcoRI-signal sequence-VL-BsiWI-CL-XhoI' (see Table 15). The polynucleotides encoding the heavy chain were respectively cloned into a vector of pOptiVEC^{™}-TOPO TA Cloning Kit included in OptiCHO^{™} Antibody Express Kit (Cat no. 12762-019; Invitrogen), and the polynucleotides encoding the light chain were respectively cloned into a vector of pcDNA^{™}3.3-TOPOTA Cloning Kit(Cat no. 8300-01), using EcoRI(NEB, R0101S) and XhoI(NEB, R0146S), to establish vectors for expressing affinity matured antibodies.

The constructed vectors were amplified using a Qiagen Maxiprep kit (Cat No. 12662), and the vectors including the heavy chain and the vector including the light chain were added to 293T cells (2.5 x 10⁷) at a ratio of about 4:1 (about 80 ug:20 ug) with 360 ul of 2 M CaCl₂ and were transfected. Next, the mixture was cultured in a DMEM medium with 10% (w/v) FBS at 37°C in 5% (v/v) CO₂ conditions for 5 hours, and then cultured in a DMEM medium without FBS at 37°C in 5% (v/v) CO₂ conditions for 48 hours.

The cultured cells were centrifuged, and 100 ml of each supernatant was purified using AKTA Prime (GE healthcare). Protein A column (GE healthcare, 17-0405-03) was placed in the AKTA Prime, and the cultured solution was flowed at a flow rate of 5 ml/min and was eluted with IgG elution buffer (Thermo Scientific, 21004). The buffer was replaced with a PBS buffer, and thus final affinity-matured antibodies (hereinafter, named as h10D6-Opti-63, h10D6-Opti-64, h10D6-Opti-65, h10D6-Opti-66, h10D6-Opti-67, h10D6-Opti-71, h10D6-Opti-68, h10D6-Opti-70, h10D6-Opti-72, and h10D6-Opti-73) were purified.

**[Table 15]**

| Clone | Antibody sequence (VH) | Antibody sequence (VL) |
|---|---|---|
| h10D6-OPTI-63 | >HU2-6.6 | >10D6_VL-Hu1-2.1 |
| | QVQLQESGPGLVKPSETLSLTCAVSGYS ITSDYAWNWIRQPPGKGLEWMGKISYS GKTDYNPSLKSRSTISRDTSKNQFSLKL SSVTAADTAVYYCARGNFEGAMDYW GQGTLVTVSS(SEQ ID NO: 52) | DIQMTQSPSSLSASVGDRVTITCKASQFV STDVHWYQQKPGKAPKLLIYYASNRYP GVPSRFSGSGSGTDFTLTISSLQPEDFATY YCQQDYSSPWTFGQGTKLEIK(SEQ ID NO: 60) |
| | (coding nucleotide sequence) | (coding nucleotide sequence) |
| | CAGGTGCAACTGCAGGAGTCAGGCCC CGGCCTGGTAAAACCTTCTGAAACGC TCTCACTTACCTGTGCCGTTAGTGGAT ACTCTATCACTTCCGACTACGCTTGGA ATTGGATTCGGCAGCCTCCAGGCAAA GGGCTGGAATGG ATGGGAAAGATTTCCTATTCCGGTAA GACTGACTACAATCCCAGTCTGAAGA GCAGGTCAACAATCTCCAGAGACACC AGCAAGAATCAGTTTTCCCTGAAATT GTCCTCGGTGACAGCAGCGGATACCG CAGTGTATTATTGCGCCCGCGGTAAC TTCGAGGGAGCTATGGATTACTGG GGGCAGGGTACTCTCGTCACTGTGAG CAGC(SEQ ID NO: 64) | GACATCCAGATGACCCAGTCTCCATCC TCCCTGTCTGCATCTGTAGGAGACAGA GTCACCATCACTTGCAAGGCCAGTCAG TTCGTGAGTACTGATGTACATTGGTAT CAGCAGAAACCAGGGAAAGCCCCTAA GCTCCTGATCTATTATGCATCCAATCGC TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 72) |
| h10D6-OPTI-64 | >HU2 -6.6 | DIQMTQSPSSLSASVGDRVTITCKASQSV |
| | QVQLQESGPGLVKPSETLSLTCAVSGYS ITSDYAWNWIRQPPGKGLEWMGKISYS GKTDYNPSLKSRSTISRDTSKNQFSLKL SSVTAADTAVYYCARGNFEGAMDYW GQGTLVTVSS(SEQ ID NO: 52) | SNDVAWYQQKPGKAPKLLIYYASNRYP GVPSRFSGSGSGTDFTLTISSLQPEDFATY YCGQDYASPWTFGQGTKLEIK(SEQ ID NO: 87) |
| | (coding nucleotide sequence) | (coding nucleotide sequence) |
| | CAGGTGCAACTGCAGGAGTCAGGCCC CGGCCTGGTAAAACCTTCTGAAACGC TCTCACTTACCTGTGCCGTTAGTGGAT ACTCTATCACTTCCGACTACGCTTGGA ATTGGATTCGGCAGCCTCCAGGCAAA GGGCTGGAATGG ATGGGAAAGATTTCCTATTCCGGTAA GACTGACTACAATCCCAGTCTGAAGA GCAGGTCAACAATCTCCAGAGACACC AGCAAGAATCAGTTTTCCCTGAAATT GTCCTCGGTGACAGCAGCGGATACCG CAGTGTATTATTGCGCCCGCGGTAAC TTCGAGGGAGCTATGGATTACTGG GGGCAGGGTACTCTCGTCACTGTGAG CAGC(SEQ ID NO: 64) | GACATCCAGATGACCCAGTCTCCATCC TCCCTGTCTGCATCTGTAGGAGACAGA GTCACCATCACTTGCAAGGCCAGTCAG AGTGTGAGTAATGATGTAGCT TGGTATCAGCAGAAACCAGGGAAAGC CCCTAAGCTCCTGATCTATTATGCATCC AACCGATACCCTGGGGTCCCATCAAGG TTCAGTGGCAGTGGATCTGGGACAGAT TTCACTCTCACCATCAGCAGTCTGCAA CCTGAAGATTTTGCAACTTACTACTGT GGACAGGATTATGCCTCTCCGTGGACG TTCGGTGGAGGCACCAAGGTGGAAATC AAA(SEQ ID NO: 88) |
| h10D6-OPTI-65 | >HU2 -6.6 | >10D6_VL-Hu1-2.4 |
| | QVQLQESGPGLVKPSETLSLTCAVSGYS ITSDYAWNWIRQPPGKGLEWMGKISYS GKTDYNPSLKSRSTISRDTSKNQFSLKL SSVTAADTAVYYCARGNFEGAMDYW GQGTLVTVSS(SEQ ID NO: 52) | DIQMTQSPSSLSASVGDRVTITCKASQSV SNDVAWYQQKPGKAPKLLIYYASIPYPG VPSRFSGSGSGTDFTLTISSLQPEDFATYY CQQDYSSPWTFGQGTKLEIK(SEQ ID NO: 61) |
| | (coding nucleotide sequence) | (coding nucleotide sequence) |
| | CAGGTGCAACTGCAGGAGTCAGGCCC CGGCCTGGTAAAACCTTCTGAAACGC TCTCACTTACCTGTGCCGTTAGTGGAT ACTCTATCACTTCCGACTACGCTTGGA ATTGGATTCGGCAGCCTCCAGGCAAA GGGCTGGAATGG ATGGGAAAGATTTCCTATTCCGGTAA GACTGACTACAATCCCAGTCTGAAGA GCAGGTCAACAATCTCCAGAGACACC AGCAAGAATCAGTTTTCCCTGAAATT GTCCTCGGTGACAGCAGCGGATACCG CAGTGTATTATTGCGCCCGCGGTAAC TTCGAGGGAGCTATGGATTACTGG GGGCAGGGTACTCTCGTCACTGTGAG CAGC(SEQ ID NO: 64) | GACATCCAGATGACCCAGTCTCCATCC TCCCTGTCTGCATCTGTAGGAGACAGA GTCACCATCACTTGCAAGGCCAGTCAG AGTGTGAGTAATGATGTAGCTTGGTAT CAGCAGAAACCAGGGAAAGCCCCTAA GCTCCTGATCTATTATGCATCCATCCCA TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 73) |
| h10D6-OPTI-66 | >HU2 -6.6 | DIQMTQSPSSLSASVGDRVTITCKASQSV SNDVHWYQQKPGKAPKLLIYYASNRYP GVPSRFSGSGSGTDFTLTISSLQPEDFATY YCGQDYSAPWTFGQGTKLEIK(SEQ ID NO: 89) |
| | QVQLQESGPGLVKPSETLSLTCAVSGYS ITSDYAWNWIRQPPGKGLEWMGKISYS GKTDYNPSLKSRSTISRDTSKNQFSLKL SSVTAADTAVYYCARGNFEGAMDYW GQGTLVTVSS(SEQ ID NO: 52) | |
| | (coding nucleotide sequence) | (coding nucleotide sequence) |
| | CAGGTGCAACTGCAGGAGTCAGGCCC CGGCCTGGTAAAACCTTCTGAAACGC TCTCACTTACCTGTGCCGTTAGTGGAT ACTCTATCACTTCCGACTACGCTTGGA ATTGGATTCGGCAGCCTCCAGGCAAA GGGCTGGAATGG | GACATCCAGATGACCCAGTCTCCATCC TCCCTGTCTGCATCTGTAGGAGACAGA GTCACCATCACTTGCAAGGCCAGTCAG AGTGTGAGTAATGATGTACAC TGGTATCAGCAGAAACCAGGGAAAGC CCCTAAGCTCCTGATCTATTATGCATCC |
| | ATGGGAAAGATTTCCTATTCCGGTAA GACTGACTACAATCCCAGTCTGAAGA GCAGGTCAACAATCTCCAGAGACACC AGCAAGAATCAGTTTTCCCTGAAATT GTCCTCGGTGACAGCAGCGGATACCG CAGTGTATTATTGCGCCCGCGGTAAC TTCGAGGGAGCTATGGATTACTGG GGGCAGGGTACTCTCGTCACTGTGAG CAGC(SEQ ID NO: 64) | AACCGATACCCTGGGGTCCCATCAAGG TTCAGTGGCAGTGGATCTGGGACAGAT TTCACTCTCACCATCAGCAGTCTGCAA CCTGAAGATTTTGCAACTTACTACTGT GGACAGGATTATTCTGCCCCGTGGACG TTCGGTGGAGGCACCAAGGTGGAAATC AAA(SEQ ID NO: 90) |
| h10D6-OPTI-67 | >HU2 -6.6 | >10D6_**VL-Hu1-2.7** |
| | QVQLQESGPGLVKPSETLSLTCAVSGYS ITSDYAWNWIRQPPGKGLEWMGKISYS GKTDYNPSLKSRSTISRDTSKNQFSLKL SSVTAADTAVYYCARGNFEGAMDYW GQGTLVTVSS(SEQ ID NO: 52) | DIQMTQSPSSLSASVGDRVTITCKASQSV SNDVHWYQQKPGKAPKLLIYYASIPYPG VPSRFSGSGSGTDFTLTISSLQPEDFATYY CQQDYSSPWTFGQGTKLEIK(SEQ ID NO: 62) |
| | (coding nucleotide sequence) | (coding nucleotide sequence) |
| | CAGGTGCAACTGCAGGAGTCAGGCCC CGGCCTGGTAAAACCTTCTGAAACGC TCTCACTTACCTGTGCCGTTAGTGGAT ACTCTATCACTTCCGACTACGCTTGGA ATTGGATTCGGCAGCCTCCAGGCAAA GGGCTGGAATGG ATGGGAAAGATTTCCTATTCCGGTAA GACTGACTACAATCCCAGTCTGAAGA GCAGGTCAACAATCTCCAGAGACACC AGCAAGAATCAGTTTTCCCTGAAATT GTCCTCGGTGACAGCAGCGGATACCG CAGTGTATTATTGCGCCCGCGGTAAC TTCGAGGGAGCTATGGATTACTGG GGGCAGGGTACTCTCGTCACTGTGAG CAGC(SEQ ID NO: 64) | GACATCCAGATGACCCAGTCTCCATCC TCCCTGTCTGCATCTGTAGGAGACAGA GTCACCATCACTTGCAAGGCCAGTCAG AGTGTGAGTAATGATGTACATTGGTAT CAGCAGAAACCAGGGAAAGCCCCTAA GCTCCTGATCTATTATGCATCCATCCCA TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 74) |
| h10D6-OPTI-71 | >HU2 -6.6 | 10D6_VL-Hu1-2.8 |
| | QVQLQESGPGLVKPSETLSLTCAVSGYS ITSDYAWNWIRQPPGKGLEWMGKISYS GKTDYNPSLKSRSTISRDTSKNQFSLKL SSVTAADTAVYYCARGNFEGAMDYW GQGTLVTVSS(SEQ ID NO: 52) | DIQMTQSPSSLSASVGDRVTITCKASQFV STDVHWYQQKPGKAPKLLIYYASIPYPG VPSRFSGSGSGTDFTLTISSLQPEDFATYY CQQDYSSPWTFGQGTKLEIK(SEQ ID NO: 63) |
| | (coding nucleotide sequence) | (coding nucleotide sequence) |
| | CAGGTGCAACTGCAGGAGTCAGGCCC CGGCCTGGTAAAACCTTCTGAAACGC TCTCACTTACCTGTGCCGTTAGTGGAT ACTCTATCACTTCCGACTACGCTTGGA ATTGGATTCGGCAGCCTCCAGGCAAA GGGCTGGAATGG ATGGGAAAGATTTCCTATTCCGGTAA GACTGACTACAATCCCAGTCTGAAGA GCAGGTCAACAATCTCCAGAGACACC AGCAAGAATCAGTTTTCCCTGAAATT GTCCTCGGTGACAGCAGCGGATACCG CAGTGTATTATTGCGCCCGCGGTAAC TTCGAGGGAGCTATGGATTACTGG GGGCAGGGTACTCTCGTCACTGTGAG CAGC(SEQ ID NO: 64) | GACATCCAGATGACCCAGTCTCCATCC TCCCTGTCTGCATCTGTAGGAGACAGA GTCACCATCACTTGCAAGGCCAGTCAG TTCGTGAGTACTGATGTACATTGGTAT CAGCAGAAACCAGGGAAAGCCCCTAA GCTCCTGATCTATTATGCATCCATCCCA TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 75) |
| h10D6-OPTI-68 | >HU3-6.6 | >10D6_VL-Hu1-2.1 |
| | EVQLVESGGGLVQPGGSLRLSCAASGY SITSDYAWNWVRQAPGKGLEWMGKIS | DIQMTQSPSSLSASVGDRVTITCKASQFV STDVHWYQQKPGKAPKLLIYYASNRYP |
| | YSGKTDYNPSLKSRSTISRDTSKNTFYL QMNSLRAEDTAVYYCARGNFEGAMD YWGQGTLVTVSS(SEQ ID NO: 54) | GVPSRFSGSGSGTDFTLTISSLQPEDFATY YCQQDYSSPWTFGQGTKLEIK(SEQ ID NO: 60) |
| | (coding nucleotide sequence) | (coding nucleotide sequence) |
| | GAGGTTCAGCTGGTCGAAAGCGGTGG GGGACTCGTGCAGCCAGGCGGTTCTC TTAGATTATCATGTGCCGCATCCGGGT ACTCCATCACCTCTGATTATGCATGGA ACTGGGTCAGACAAGCCCCCGGAAAG GGCCTGGAGTGGATGGGGAAGATCTC CTATTCAGGGAAGACAGATTATAATC CTTCGCTGAAAAGCAGATCAACAATT AGTAGAGACACTTCTAAAAATACTTT TTACCTCCAGATGAACAGTCTGCGCG CCGAAGACACCGCCGTGTACTACTGC GCTAGGGGAAATTTCGAGGGAGCTAT GGACTATTGGGGCCAGGGCACGTTGG TAACCGTGAGCAGC(SEQ ID NO: 66) | GACATCCAGATGACCCAGTCTCCATCC TCCCTGTCTGCATCTGTAGGAGACAGA GTCACCATCACTTGCAAGGCCAGTCAG TTCGTGAGTACTGATGTACATTGGTAT CAGCAGAAACCAGGGAAAGCCCCTAA GCTCCTGATCTATTATGCATCCAATCGC TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 72) |
| h10D6-OPTI-70 | >HU3-6.6 | >10D6_VL-Hu1-2.4 |
| | EVQLVESGGGLVQPGGSLRLSCAASGY SITSDYAWNWVRQAPGKGLEWMGKIS YSGKTDYNPSLKSRSTISRDTSKNTFYL QMNSLRAEDTAVYYCARGNFEGAMD YWGQGTLVTVSS(SEQ ID NO: 54) | DIQMTQSPSSLSASVGDRVTITCKASQSV SNDVAWYQQKPGKAPKLLIYYASIPYPG VPSRFSGSGSGTDFTLTISSLQPEDFATYY CQQDYSSPWTFGQGTKLEIK(SEQ ID NO: 61) |
| | (coding nucleotide sequence) | (coding nucleotide sequence) |
| | GAGGTTCAGCTGGTCGAAAGCGGTGG GGGACTCGTGCAGCCAGGCGGTTCTC TTAGATTATCATGTGCCGCATCCGGGT ACTCCATCACCTCTGATTATGCATGGA ACTGGGTCAGACAAGCCCCCGGAAAG GGCCTGGAGTGGATGGGAAGATCTC CTATTCAGGGAAGACAGATTATAATC CTTCGCTGAAAAGCAGATCAACAATT AGTAGAGACACTTCTAAAAATACTTT TTACCTCCAGATGAACAGTCTGCGCG CCGAAGACACCGCCGTGTACTACTGC GCTAGGGGAAATTTCGAGGGAGCTAT GGACTATTGGGGCCAGGGCACGTTGG TAACCGTGAGCAGC(SEQ ID NO: 66) | GACATCCAGATGACCCAGTCTCCATCC TCCCTGTCTGCATCTGTAGGAGACAGA GTCACCATCACTTGCAAGGCCAGTCAG AGTGTGAGTAATGATGTAGCTTGGTAT CAGCAGAAACCAGGGAAAGCCCCTAA GCTCCTGATCTATTATGCATCCATCCCA TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 73) |
| h10D6-OPTI-72 | >HU3-6.6 | >10D6_VL-Hu1-2.7 |
| | EVQLVESGGGLVQPGGSLRLSCAASGY SITSDYAWNWVRQAPGKGLEWMGKIS YSGKTDYNPSLKSRSTISRDTSKNTFYL QMNSLRAEDTAVYYCARGNFEGAMD YWGQGTLVTVSS(SEQ ID NO: 54) | DIQMTQSPSSLSASVGDRVTITCKASQSV SNDVHWYQQKPGKAPKLLIYYASIPYPG VPSRFSGSGSGTDFTLTISSLQPEDFATYY CQQDYSSPWTFGQGTKLEIK(SEQ ID NO: 62) |
| | (coding nucleotide sequence) | (coding nucleotide sequence) |
| | GAGGTTCAGCTGGTCGAAAGCGGTGG GGGACTCGTGCAGCCAGGCGGTTCTC TTAGATTATCATGTGCCGCATCCGGGT ACTCCATCACCTCTGATTATGCATGGA ACTGGGTCAGACAAGCCCCCGGAAAG GGCCTGGAGTGGATGGGGAAGATCTC CTATTCAGGGAAGACAGATTATAATC CTTCGCTGAAAAGCAGATCAACAATT AGTAGAGACACTTCTAAAAATACTTT TTACCTCCAGATGAACAGTCTGCGCG | GACATCCAGATGACCCAGTCTCCATCC TCCCTGTCTGCATCTGTAGGAGACAGA GTCACCATCACTTGCAAGGCCAGTCAG AGTGTGAGTAATGATGTACATTGGTAT CAGCAGAAACCAGGGAAAGCCCCTAA GCTCCTGATCTATTATGCATCCATCCCA TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG |
| | CCGAAGACACCGCCGTGTACTACTGC GCTAGGGGAAATTTCGAGGGAGCTAT GGACTATTGGGGCCAGGGCACGTTGG TAACCGTGAGCAGC(SEQ ID NO: 66) | GATTATAGCTCTCCGTGGACGTTCGGT GGAGGCACCAAGGTGGAAATCAAA(SE Q ID NO: 74) |
| h10D6-OPTI-73 | >HU3-6.6 | 10D6_VL-Hu1-2.8 |
| | EVQLVESGGGLVQPGGSLRLSCAASGY SITSDYAWNWVRQAPGKGLEWMGKIS YSGKTDYNPSLKSRSTISRDTSKNTFYL QMNSLRAEDTAVYYCARGNFEGAMD YWGQGTLVTVSS(SEQ ID NO: 54) | DIQMTQSPSSLSASVGDRVTITCKASQFV STDVHWYQQKPGKAPKLLIYYASIPYPG VPSRFSGSGSGTDFTLTISSLQPEDFATYY CQQDYSSPWTFGQGTKLEIK(SEQ ID NO: 63) |
| | (coding nucleotide sequence) | (coding nucleotide sequence) |
| | GAGGTTCAGCTGGTCGAAAGCGGTGG GGGACTCGTGCAGCCAGGCGGTTCTC TTAGATTATCATGTGCCGCATCCGGGT ACTCCATCACCTCTGATTATGCATGGA ACTGGGTCAGACAAGCCCCCGGAAAG GGCCTGGAGTGGATGGGGAAGATCTC CTATTCAGGGAAGACAGATTATAATC CTTCGCTGAAAAGCAGATCAACAATT AGTAGAGACACTTCTAAAAATACTTT TTACCTCCAGATGAACAGTCTGCGCG CCGAAGACACCGCCGTGTACTACTGC GCTAGGGGAAATTTCGAGGGAGCTAT GGACTATTGGGGCCAGGGCACGTTGG TAACCGTGAGCAGC(SEQ ID NO: 66) | GACATCCAGATGACCCAGTCTCCATCC TCCCTGTCTGCATCTGTAGGAGACAGA GTCACCATCACTTGCAAGGCCAGTCAG TTCGTGAGTACTGATGTACATTGGTAT CAGCAGAAACCAGGGAAAGCCCCTAA GCTCCTGATCTATTATGCATCCATCCCA TACCCTGGGGTCCCATCAAGGTTCAGT GGCAGTGGATCTGGGACAGATTTCACT CTCACCATCAGCAGTCTGCAACCTGAA GATTTTGCAACTTACTACTGTCAGCAG GATTATAGCTCTCCGTGGACGTTCGGT GGAGGCACCAAGGTGGAAATCAAA(SE Q ID NO: 75) |

(In Table 15, the bold letters are CDR1, CDR2, and CDR3 in sequence)

### Example 13: Analysis of binding affinity of selected antibodies

The binding affinity (KD values) of the antibodies to human Ang2 protein was measured by an SPR method using a BIAcore T100 (GE Healthcare). 25 µg/ml anti-His antibody was immobilized on a CM5 sensor chip (GE healthcare) using a pH 5.0 acetate solution and an amine coupling kit (GE Healthcare). 6 µg/ml of a recombinant hAng2 (C-His, R&D Systems) protein was flowed onto the chip to be captured at 100 to 200 RU levels. The antibodies obtained in the above examples were diluted serially to twice each time starting from 100 nM concentration and it was each flowed onto the chip to allow it to be bound to (on), dissociated from (off), and regenerated (using 10 mM NaOH solution) from the antigen captured on the sensor chip, thereby to measure antigen-antibody affinity. The KD values were calculated from the values of kₒₙ k_{off}, and the results are as shown in the following Table 16.

**[Table 16]**

| Antibody | kon (1/Ms) | koff (1/s) | KD (M) |
|---|---|---|---|
| h10D6-OPTI-63 | 2.676x10⁶ | 7.421x10⁻⁵ | 2.773x10⁻¹¹ |
| h10D6-OPTI-65 | 4.960x16⁵ | 2.250x10⁻⁵ | 4.536x10⁻¹² |
| h10D6-OPTI-67 | 2.080x16⁶ | 2.684x10⁻⁷ | 1.291x10⁻¹³ |
| h10D6-OPTI-68 | 5.355x10⁵ | 1.696x10⁻⁴ | 3.168x10⁻¹⁰ |
| h10D6-OPTI-70 | 2.650x10⁵ | 1.159x10⁻⁴ | 4.374x10⁻¹⁰ |

As shown in Table 16, all the the affinity-matured and humanized antibodies show high affinity to Ang2 from about 0.000129nM to about 0.43nM.

### EXAMPLE 14: Preventive Effect of Anti-Ang2 Antibody against Thymus involution as Verified by Thymus Size

Thymus involution is linked to senescence, and is found abundantly in patients with immune-related diseases such as acquired immune deficiency syndrome. To examine the effect of the anti-Ang2 antibody on thymus involution, the following experiments were preformed. Of the anti-Ang2 antibodies that were selected for affinity in Examples 1 to 13, the h10D6-Opti-67 antibody was illustratively used.

Mice at 4 months (young) and 24 months (old) of age were intraperitoneally injected once a week with an anti-Ang2 antibody (h10D6-Opti-67) or a control antibody (human IgG1) at a dose of 4 mg/kg for 8 weeks. Young and old mice injected with the control antibody were expressed as YC and OC, respectively, while the old mice injected with the anti-Ang2 antibody were denoted as OA. Eight weeks after antibody injection, the thymuses were isolated from the controls (YC, OC) and the experimental group (OA) and measured for morphology and size.

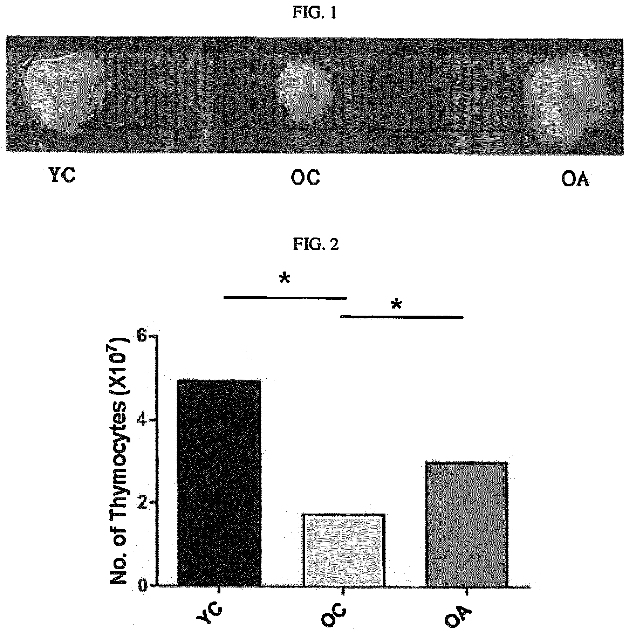

The results are depicted in Fig. 1. As can be seen in Fig. 1, the control antibody-treated old mice (OC) showed a reduction in thymus size of 50 % or greater compared to the control antibody-treated young mice (YC), whereas the thymi from the old mice injected with the anti-Ang2 antibody (OA) were almost as large as those from the control young mice. These results indicate that the anti-Ang2 antibody is effective for preventing thymus involution.

### EXAMPLE 15: Preventive Effect of Anti-Ang2 Antibody against Thymus involution as Verified by Thymocyte Count

Thymus involution caused by senescence or immune-related diseases comprising AIDS causes a decrease in thymocyte count (number of hematopoietic stem cells in the thymus). In order to examine the effect of the anti-Ang2 antibody on thymocyte count, thymocytes were collected from the thymuses of the same mice as in Example 14 (YC, OC, OA; intraperitoneally injected once a week with the h10D6-Opti-67 antibody or the control antibody in a dose of 4 mg/kg for 8 weeks) and counted.

The results are depicted in Fig. 2 (mean values of experiments run in triplicate). As can be seen in Fig. 2, the control antibody-treated old mice (OC) were greatly reduced in thymocyte count compared to the control antibody-treated young mice (YC), whereas the old mice injected with the anti-Ang2 antibody (OA) showed a statistically significant increase in thymocyte count.

### EXAMPLE 16: Ameliorative Effect of Anti-Ang2 Antibody on Thymic Function

Spleens were isolated from the same mouse groups as in Example 14 (YC, OC, OA; intraperitoneally injected once a week with the h10D6-Opti-67 antibody or the control antibody at a dose of 4 mg/kg for 8 weeks), and analyzed for the level of naive CD4+ T cells, which play an important role in the implementation of thymic functions, by flow cytometry (LSRFortess flow cytometry, BD) using CD4, CD3, CD44, and CD62L markers (antibodies; all purchased from Abcam).

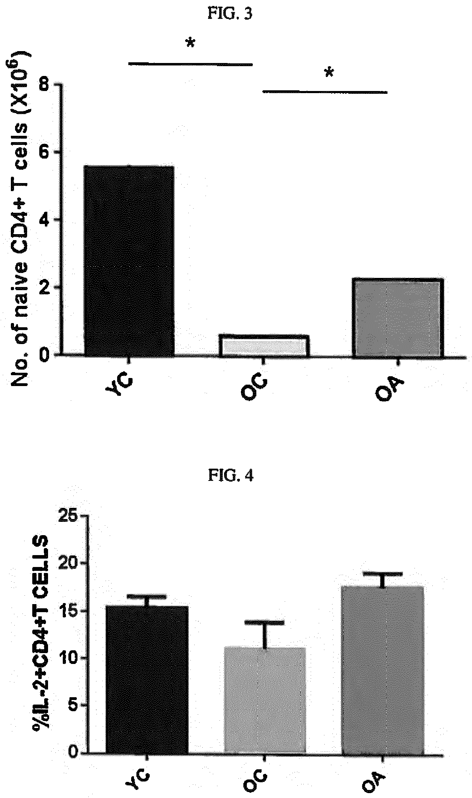

The results are depicted in Fig. 3 (mean values of experiments run in triplicate). As is understood from the data of Fig. 3, a sharp increase in the naïve CD4+T cell count was detected in the control antibody-treated old mice (OC) compared to the control antibody-treated young mice (YC), whereas an increase was observed in the anti-Ang2 antibody-treated old mice (OA), demonstrating that the anti-Ang2 antibody ameliorates thymic function.

### EXAMPLE 17: Restorative Effect of Anti-Ang2 Antibody on Immune Function (Peripheral T Cell Function)

The IL-2 expression of CD4+ T cells is down-regulated with senescence or in patients with immune-related diseases such as AIDS. IL-2 is representative of the cytokines that promote the proliferation of lymphocytes to thus enhance immune function.

Levels of CD4+ T cells secreting the cytokine IL-2 in the same mouse groups as in Example 14 (YC, OC, OA; intraperitoneally injected once a week with the h10D6-Opti-67 antibody or the control antibody at a dose of 4 mg/kg for 8 weeks) were analyzed. Following immunostaining with anti-IL-2 antibody (Abcam), IL-2+, CD4+ T cells were counted via flow cytometry (LSRFortess flow cytometry, BD).

Ratios of the IL-2-secreating CD4+ T cells (%; No of IL-2+, CD4+ T cells / No of total CD4+ T cells) are depicted in Fig. 4. As shown in Fig. 4, the ability of CD4+ T cells to produce IL-2 was degraded in the control antibody-treated old mice (OC) compared to the control antibody-treated young mice (YC), but was restored in the anti-Ang2 antibody-treated old mice (OA).

### EXAMPLE 18: Promotive Effect of Anti-Ang2 Antibody on Self-Renewal of Hematopoietic Stem Cells

Myelocytes were isolated from the hind limb bones of the same mouse groups as in Example 14 (YC, OC, OA; intraperitoneally injected once a week with the h10D6-Opti-67 antibody or the control antibody in a dose of 4 mg/kg for 8 weeks) and were measured for % of hematopoietic stem cells therein by flow cytometry (LSRFortess flow cytometry, BD). Particularly, the percentage of self-renewal hematopoietic stem cells (long-term hematopoietic stem cells; LT-HSC), which is a main factor for T cell differentiation, was measured by immunostaining using lineage negative (CD3-, B220-, CD11b-, Ter119-, Gr1-), c-Kit, Flk2, and CD34 makers (Abcam).

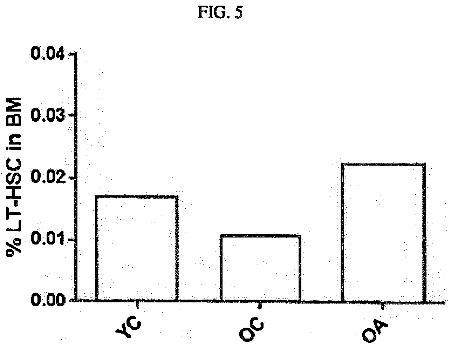

The measured ratios of self-renewal hematopoietic stem cells (%; No. of long-term hematopoietic stem cells / No. of total hematopoietic stem cells) are depicted in Fig. 5 (mean values of experiments run in triplicate). As shown in Fig. 5, the level of self-renewal hematopoietic stem cells was increased in the anti-Ang2 antibody-treated old mice (OA), compared to the control antibody-treated groups (YC, OC).

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. An anti-Ang2 antibody or an antigen-binding fragment thereof for use in potentiating immunity, wherein the anti-Ang2 antibody or antigen-binding fragment thereof binds to Ang2 and forms a complex with Tie2 receptor through Ang2.

2. The anti-Ang2 antibody or an antigen-binding fragment thereof for use of claim 1, wherein the anti-Ang2 antibody or antigen-binding fragment thereof binds to Q418, P419, a combination of Q418 and P419, or 2 to 20 consecutive acid residues of human Ang2 of SEQ ID NO: 11, including Q418, P419, a combination of Q418 or P419.

3. The anti-Ang2 antibody or an antigen-binding fragment thereof for use of claim 1 or 2, wherein the anti-Ang2 antibody or antigen-binding fragment thereof comprises:
a heavy chain complementarity-determining region H1 (CDR-H1) comprising SEQ ID NO: 1, a CDR-H2 comprising SEQ ID NO: 20, and a CDR-H3 comprising SEQ ID NO: 3;
a light heavy chain complementarity-determining region-L1 (CDR-L1) comprising SEQ ID NO: 21, a CDR-L2 comprising SEQ ID NO: 22, and a CDR-L3 comprising SEQ ID NO: 23.

4. The anti-Ang2 antibody or an antigen-binding fragment thereof for use of claim 3, wherein the anti-Ang2 antibody or antigen-binding fragment thereof comprises:
a CDR-H1 comprising SEQ ID NO: 1, a CDR-H2 comprising SEQ ID NO: 2, 14 or 15, and a CDR-H3 comprising SEQ ID NO: 3;
a CDR-L1 comprising SEQ ID NO: 4, 16, or 17, a CDR-L2 comprising SEQ ID NO: 5 or 18, and a CDR-L3 comprising SEQ ID NO: 6 or 19.

5. The anti-Ang2 antibody or an antigen-binding fragment thereof for use of claim 4, wherein the anti-Ang2 antibody or antigen-binding fragment thereof comprises:
a heavy chain variable region comprising SEQ ID NO: 7, 52, 53, 54, 55, or 56; and
a light chain variable region comprising SEQ ID NO: 8, 57, 58, 59, 60, 61, 62, 63, 87, or 89.

6. The anti-Ang2 antibody or an antigen-binding fragment thereof for use of any one of claims 1 to 5, wherein the anti-Ang2 antibody or antigen-binding fragment thereof is a mouse antibody, a chimeric antibody, or a humanized antibody.

7. The anti-Ang2 antibody or an antigen-binding fragment thereof for use of any one of claims 1 to 6, wherein the anti-Ang2 antibody or an antigen-binding fragment thereof is used in 1) improvement or increase in resistance to thymus involution, thymic function, the number of T cells, or production of hematopoietic stem cells, or 2) augmentation or recovery of T cell functions, and the like

8. An anti-Ang2 antibody or an antigen-binding fragment thereof for use in preventing or treating an immune-related disease, comprising administering an anti-Ang2 antibody or an antigen-binding fragment thereof to a subject in need of preventing or treating an immune-related disease, wherein the anti-Ang2 antibody or antigen-binding fragment thereof binds to Ang2 and forms a complex with Tie2 receptor through Ang2.

9. The anti-Ang2 antibody or an antigen-binding fragment thereof for use of claim 8, wherein the anti-Ang2 antibody or antigen-binding fragment thereof binds to Q418, P419, a combination of Q418 and P419, or 2 to 20 consecutive acid residues of human Ang2 of SEQ ID NO: 11, including Q418, P419, a combination of Q418 or P419.

10. The anti-Ang2 antibody or an antigen-binding fragment thereof for use of claim 8 or 9, wherein the anti-Ang2 antibody or antigen-binding fragment thereof comprises:
a CDR-H1 comprising SEQ ID NO: 1, a CDR-H2 comprising SEQ ID NO: 20, and a CDR-H3 comprising SEQ ID NO: 3;
a CDR-L1 comprising SEQ ID NO: 21, a CDR-L2 comprising SEQ ID NO: 22, and a CDR-L3 comprising SEQ ID NO: 23.

11. The anti-Ang2 antibody or an antigen-binding fragment thereof for use of claim 10, wherein the anti-Ang2 antibody or antigen-binding fragment thereof comprises:
a CDR-H1 comprising SEQ ID NO: 1, a CDR-H2 comprising SEQ ID NO: 2, 14 or 15, and a CDR-H3 comprising SEQ ID NO: 3;
a CDR-L1 comprising SEQ ID NO: 4, 16, or 17, a CDR-L2 comprising SEQ ID NO: 5 or 18, and a CDR-L3 comprising SEQ ID NO: 6 or 19.

12. The anti-Ang2 antibody or an antigen-binding fragment thereof for use of claim 11, wherein the anti-Ang2 antibody or antigen-binding fragment thereof comprises:
a heavy chain variable region comprising SEQ ID NO: 7, 52, 53, 54, 55, or 56; and
a light chain variable region comprising SEQ ID NO: 8, 57, 58, 59, 60, 61, 62, 63, 87, or 89.

13. The anti-Ang2 antibody or an antigen-binding fragment thereof for use of any one of claims 8 to 12, wherein the anti-Ang2 antibody or antigen-binding fragment thereof is a mouse antibody, a chimeric antibody, or a humanized antibody.

14. The anti-Ang2 antibody or an antigen-binding fragment thereof for use of any one of claims 8 to 13, wherein the immune-related disease is acquired immune deficiency syndrome or an autoimmune disease.


<110>    SAMSUNG ELECTRONICS CO., LTD.

<120>    Immunopotentiator containing anti-Ang2 antibody

<130>    EP110109FZ200pau

<140>    not yet assigned
<141>    herewith

<150>    KR10-2015-0186457
<151>    2015-12-24

<160>    90

<170>    KopatentIn 1.71

<210>    1
<211>    6
<212>    PRT
<213>    Artificial Sequence

<220>
<223>    CDR-H1 of anti-Ang2 antibody


<400>    1
Ser Asp Tyr Ala Trp Asn
  1               5


<210>    2
<211>    16
<212>    PRT
<213>    Artificial Sequence

<220>
<223>    CDR-H2 of anti-Ang2 antibody


<400>    2
Tyr Ile Asn Tyr Ser Gly Asn Thr Asp Tyr Asn Pro Ser Leu Lys Ser
  1               5                  10                  15




<210>    3
<211>    9
<212>    PRT
<213>    Artificial Sequence

<220>
<223>    CDR-H3 of anti-Ang2 antibody


<400>    3
Gly Asn Phe Glu Gly Ala Met Asp Tyr
  1               5


<210>    4
<211>    11
<212>    PRT

<213>    Artificial Sequence

<220>
<223>    CDR-L1 of anti-Ang2 antibody


<400>    4
Lys Ala Ser Gln Ser Val Ser Asn Asp Val Ala
1               5                   10


<210>    5
<211>    7
<212>    PRT
<213>    Artificial Sequence

<220>
<223>    CDR-L2 of anti-Ang2 antibody


<400>    5
Tyr Ala Ser Asn Arg Tyr Pro
1               5


<210>    6
<211>    9
<212>    PRT
<213>    Artificial Sequence

<220>
<223>    CDR-L3 of anti-Ang2 antibody


<400>    6
Gln Gln Asp Tyr Ser Ser Pro Trp Thr
1               5


<210>    7
<211>    118
<212>    PRT
<213>    Artificial Sequence

<220>
<223>    Amino acid sequence of heavy chain variable region


<400>    7
Asp Val Gln Leu Gln Glu Ser Gly Pro Asp Leu Val Lys Pro Ser Gln
1               5                   10                  15

Ser Leu Ser Leu Thr Cys Thr Val Thr Gly Tyr Ser Ile Thr Ser Asp
            20                  25                  30

Tyr Ala Trp Asn Trp Ile Arg Gln Phe Pro Gly Asn Lys Leu Glu Trp
        35                  40                  45

Met Gly Tyr Ile Asn Tyr Ser Gly Asn Thr Asp Tyr Asn Pro Ser Leu
    50                  55                  60

Lys Ser Arg Ser Ser Ile Thr Arg Asp Thr Ser Lys Asn Gln Phe Phe
65                  70                  75                  80

Leu Gln Leu Asn Ser Val Thr Thr Gly Asp Thr Ala Thr Tyr Tyr Cys
                85                  90                  95

Ala Arg Gly Asn Phe Glu Gly Ala Met Asp Tyr Trp Gly Gln Gly Thr
            100                 105                 110

Ser Val Thr Val Ser Ser
        115


<210>    8
<211>    354
<212>    DNA
<213>    Artificial Sequence

<220>
<223>    Nucleotide sequence encoding heavy chain variable region


<400>    8
gatgtgcagc ttcaggagtc gggacctgac ctggtgaaac cttctcagtc tctgtccctc          60

acctgcactg tcactggcta ctcaatcacc agtgattatg cctggaactg gatccggcag         120

tttccaggaa acaaactgga gtggatgggc tacataaact acagtggtaa cactgactac         180

aacccatctc tcaaaagtcg aagctctatc actcgagaca catccaagaa ccagttcttc         240

ctgcagttga attctgtgac tactggggac acagccacat attactgtgc aagaggtaac         300

ttcgaaggtg ctatggacta ctggggtcaa ggaacctcag tcaccgtctc ctca               354


<210>    9
<211>    107
<212>    PRT
<213>    Artificial Sequence

<220>
<223>    Amino acid sequence of light chain variable region


<400>    9
Ser Ile Val Met Thr Gln Thr Pro Lys Phe Leu Leu Val Ser Ala Gly
  1               5                  10                  15

Asp Arg Val Thr Ile Thr Cys Lys Ala Ser Gln Ser Val Ser Asn Asp
            20                  25                  30

Val Ala Trp Tyr Gln Gln Lys Pro Gly Gln Ser Pro Lys Leu Leu Ile
        35                  40                  45

Tyr Tyr Ala Ser Asn Arg Tyr Pro Gly Val Pro Asp Arg Phe Thr Gly
    50                  55                  60

Ser Gly Tyr Gly Thr Asp Phe Thr Phe Thr Ile Ser Thr Val Gln Ala
 65                  70                  75                  80

Glu Asp Leu Ala Val Tyr Phe Cys Gln Gln Asp Tyr Ser Ser Pro Trp
                85                  90                  95

Thr Phe Gly Gly Gly Thr Lys Leu Glu Ile Lys

            100                 105

<210> 10
<211> 321
<212> DNA
<213> Artificial Sequence

<220>
<223> Nucleotide sequence emcoding light chain variable region

<400> 10
agtattgtga tgacccagac tcccaaattc ctgcttgtat cagcaggaga cagggttacc 60

ataacctgca aggccagtca gagtgtgagt aatgatgtag cttggtacca acagaagcca 120

gggcagtctc ctaaactgct gatatactat gcatccaatc gctaccctgg agtccctgat 180

cgcttcactg gcagtggata tgggacggat ttcactttca ccatcagcac tgtgcaggct 240

gaagacctgg cagtttattt ctgtcagcag gattatagct ctccgtggac gttcggtgga 300

ggcaccaagc tggaaatcaa a 321

<210> 11
<211> 496
<212> PRT
<213> Artificial Sequence

<220>
<223> Human Ang2

<400> 11
Met Trp Gln Ile Val Phe Phe Thr Leu Ser Cys Asp Leu Val Leu Ala
1               5                   10                  15

Ala Ala Tyr Asn Asn Phe Arg Lys Ser Met Asp Ser Ile Gly Lys Lys
            20                  25                  30

Gln Tyr Gln Val Gln His Gly Ser Cys Ser Tyr Thr Phe Leu Leu Pro
        35                  40                  45

Glu Met Asp Asn Cys Arg Ser Ser Ser Ser Pro Tyr Val Ser Asn Ala
    50                  55                  60

Val Gln Arg Asp Ala Pro Leu Glu Tyr Asp Asp Ser Val Gln Arg Leu
65                  70                  75                  80

Gln Val Leu Glu Asn Ile Met Glu Asn Asn Thr Gln Trp Leu Met Lys
                85                  90                  95

Leu Glu Asn Tyr Ile Gln Asp Asn Met Lys Lys Glu Met Val Glu Ile
            100                 105                 110

Gln Gln Asn Ala Val Gln Asn Gln Thr Ala Val Met Ile Glu Ile Gly
        115                 120                 125

Thr Asn Leu Leu Asn Gln Thr Ala Glu Gln Thr Arg Lys Leu Thr Asp
    130                 135                 140

Val Glu Ala Gln Val Leu Asn Gln Thr Thr Arg Leu Glu Leu Gln Leu
145                 150                 155                 160

Leu Glu His Ser Leu Ser Thr Asn Lys Leu Glu Lys Gln Ile Leu Asp
                165                 170                 175

Gln Thr Ser Glu Ile Asn Lys Leu Gln Asp Lys Asn Ser Phe Leu Glu
            180                 185                 190

Lys Lys Val Leu Ala Met Glu Asp Lys His Ile Ile Gln Leu Gln Ser
        195                 200                 205

Ile Lys Glu Glu Lys Asp Gln Leu Gln Val Leu Val Ser Lys Gln Asn
    210                 215                 220

Ser Ile Ile Glu Glu Leu Glu Lys Lys Ile Val Thr Ala Thr Val Asn
225                 230                 235                 240

Asn Ser Val Leu Gln Lys Gln Gln His Asp Leu Met Glu Thr Val Asn
                245                 250                 255

Asn Leu Leu Thr Met Met Ser Thr Ser Asn Ser Ala Lys Asp Pro Thr
            260                 265                 270

Val Ala Lys Glu Glu Gln Ile Ser Phe Arg Asp Cys Ala Glu Val Phe
        275                 280                 285

Lys Ser Gly His Thr Thr Asn Gly Ile Tyr Thr Leu Thr Phe Pro Asn
    290                 295                 300

Ser Thr Glu Glu Ile Lys Ala Tyr Cys Asp Met Glu Ala Gly Gly Gly
305                 310                 315                 320

Gly Trp Thr Ile Ile Gln Arg Arg Glu Asp Gly Ser Val Asp Phe Gln
                325                 330                 335

Arg Thr Trp Lys Glu Tyr Lys Val Gly Phe Gly Asn Pro Ser Gly Glu
            340                 345                 350

Tyr Trp Leu Gly Asn Glu Phe Val Ser Gln Leu Thr Asn Gln Gln Arg
        355                 360                 365

Tyr Val Leu Lys Ile His Leu Lys Asp Trp Glu Gly Asn Glu Ala Tyr
    370                 375                 380

Ser Leu Tyr Glu His Phe Tyr Leu Ser Ser Glu Glu Leu Asn Tyr Arg
385                 390                 395                 400

Ile His Leu Lys Gly Leu Thr Gly Thr Ala Gly Lys Ile Ser Ser Ile
                405                 410                 415

Ser Gln Pro Gly Asn Asp Phe Ser Thr Lys Asp Gly Asp Asn Asp Lys
            420                 425                 430

Cys Ile Cys Lys Cys Ser Gln Met Leu Thr Gly Gly Trp Trp Phe Asp
        435                 440                 445

Ala Cys Gly Pro Ser Asn Leu Asn Gly Met Tyr Tyr Pro Gln Arg Gln
    450                 455                 460

Asn Thr Asn Lys Phe Asn Gly Ile Lys Trp Tyr Tyr Trp Lys Gly Ser
465                 470                 475                 480

Gly Tyr Ser Leu Lys Ala Thr Thr Met Met Ile Arg Pro Ala Asp Phe
                485                 490                 495

<210>   12
<211>   121
<212>   PRT
<213>   Artificial Sequence

<220>
<223>   Amino acid sequence of heavy chain variable region (4H10)

<400>   12
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Asp Met Ser Trp Val Arg Gln Ala Pro Gly Lys Gly Leu Glu Trp Val
        35                  40                  45

Ser Leu Ile Ser Pro Asp Ser Ser Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Leu Ile Ser Phe Trp Arg Gly Gly Phe Asp Tyr Trp Gly
            100                 105                 110

Gln Gly Thr Leu Val Thr Val Ser Ser
        115                 120

<210>   13
<211>   111
<212>   PRT
<213>   Artificial Sequence

<220>
<223>   Amino acid sequence of light chain variable region (4H10)

<400>   13
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Asn Trp Tyr Gln Gln Leu Pro Gly Thr Ala Pro Lys Leu Leu
        35                  40                  45

Ile Tyr Ala Asp Ser Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Tyr Ser Leu
                85                  90                  95

Ser Gly Tyr Val Phe Gly Gly Gly Thr Lys Leu Thr Val Leu Gly
            100                 105                 110

<210>    14
<211>    16
<212>    PRT
<213>    Artificial Sequence

<220>
<223>    CDR-H2 of anti-Ang2 antibody

<400>    14
Lys Ile Ser Tyr Ser Gly Lys Thr Asp Tyr Asn Pro Ser Leu Lys Ser
1               5                   10                  15

<210>    15
<211>    16
<212>    PRT
<213>    Artificial Sequence

<220>
<223>    CDR-H2 of anti-Ang2 antibody

<400>    15
Lys Ile Asn Tyr Ala Gly Asn Thr Asp Tyr Asn Pro Ser Leu Lys Ser
1               5                   10                  15

<210>    16
<211>    11
<212>    PRT
<213>    Artificial Sequence

<220>
<223>    CDR-L1 of anti-Ang2 antibody

<400>    16
Lys Ala Ser Gln Ser Val Ser Asn Asp Val His
1               5                   10

<210>    17
<211>    11
<212>    PRT
<213>    Artificial Sequence

<220>
<223>    CDR-L1 of anti-Ang2 antibody


<400>    17
Lys Ala Ser Gln Phe Val Ser Thr Asp Val His
 1               5                   10


<210>    18
<211>    7
<212>    PRT
<213>    Artificial Sequence

<220>
<223>    CDR-L2 of anti-Ang2 antibody


<400>    18
Tyr Ala Ser Ile Pro Tyr Pro
 1               5


<210>    19
<211>    9
<212>    PRT
<213>    Artificial Sequence

<220>
<223>    CDR-L3 of anti-Ang2 antibody


<400>    19
Gln His Asp Tyr Ser Ser Pro Phe Thr
 1               5


<210>    20
<211>    16
<212>    PRT
<213>    Artificial Sequence

<220>
<223>    CDR-H2 of anti-Ang2 antibody


<220>
<221>    MOD_RES
<222>    (1)
<223>    X is Tyr (Y) or Lys (K)


<220>
<221>    MOD_RES
<222>    (3)
<223>    X is Asn (N) or Ser (S)


<220>
<221>    MOD_RES
<222>    (5)
<223>    X is Ser (S) or Ala (A)

<220>
<221>    MOD_RES
<222>    (7)
<223>    X is Asn (N) or Lys (K)

<400>    20
Xaa Ile Xaa Tyr Xaa Gly Xaa Thr Asp Tyr Asn Pro Ser Leu Lys Ser
1               5                   10                  15

<210>    21
<211>    11
<212>    PRT
<213>    Artificial Sequence

<220>
<223>    CDR-L1 of anti-Ang2 antibody

<220>
<221>    MOD_RES
<222>    (5)
<223>    X is Ser (S) or Phe (F)

<220>
<221>    MOD_RES
<222>    (8)
<223>    X is Asn (N) or Thr (T)

<220>
<221>    MOD_RES
<222>    (11)
<223>    X is Ala (A) or His (H)

<400>    21
Lys Ala Ser Gln Xaa Val Ser Xaa Asp Val Xaa
1               5                   10

<210>    22
<211>    7
<212>    PRT
<213>    Artificial Sequence

<220>
<223>    CDR-L2 of anti-Ang2 antibody

<220>
<221>    MOD_RES
<222>    (4)
<223>    X is Asn (N) or Ile (I)

<220>
<221>    MOD_RES

<222>    (5)
<223>    X is Arg (R) or Pro (P)


<400>    22
Tyr Ala Ser Xaa Xaa Tyr Pro
1               5


<210>    23
<211>    9
<212>    PRT
<213>    Artificial Sequence

<220>
<223>    CDR-L3 of anti-Ang2 antibody


<220>
<221>    MOD_RES
<222>    (2)
<223>    X is Gln (Q) or His (H)


<220>
<221>    MOD_RES
<222>    (8)
<223>    X is Trp (W) or Phe (F)


<400>    23
Gln Xaa Asp Tyr Ser Ser Pro Xaa Thr
1               5


<210>    24
<211>    30
<212>    PRT
<213>    Artificial Sequence

<220>
<223>    FR1 (N-terminal frame region of CDR-H1 of 10D6)


<400>    24
Asp Val Gln Leu Gln Glu Ser Gly Pro Asp Leu Val Lys Pro Ser Gln
1               5                   10                  15

Ser Leu Ser Leu Thr Cys Thr Val Thr Gly Tyr Ser Ile Thr
            20                  25                  30


<210>    25
<211>    30
<212>    PRT
<213>    Artificial Sequence

<220>
<223>    FR1 (N-terminal frame region of CDR-H1 of VH-hu1)


<400>    25
Gln Val Gln Leu Gln Glu Ser Gly Pro Gly Leu Val Lys Pro Ser Glu

1               5                   10                  15

Thr Leu Ser Leu Thr Cys Ala Val Ser Gly Tyr Ser Ile Ser
            20                  25                  30



<210>   26
<211>   30
<212>   PRT
<213>   Artificial Sequence

<220>
<223>   FR1 (N-terminal frame region of CDR-H1 of VH-hu2)


<400>   26
Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Thr
            20                  25                  30



<210>   27
<211>   30
<212>   PRT
<213>   Artificial Sequence

<220>
<223>   FR1 (N-terminal frame region of CDR-H1 of VH-hu5)


<400>   27
Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Thr
            20                  25                  30



<210>   28
<211>   30
<212>   PRT
<213>   Artificial Sequence

<220>
<223>   FR1 (N-terminal frame region of CDR-H1 of VH-hu3)


<400>   28
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Ile Thr
            20                  25                  30



<210>   29
<211>   14
<212>   PRT
<213>   Artificial Sequence

<220>

<223>    FR2 (frame region between CDR-H1 and CDR-H2 of 10D6)

<400>    29
Trp Ile Arg Gln Phe Pro Gly Asn Lys Leu Glu Trp Met Gly
1               5                   10

<210>    30
<211>    14
<212>    PRT
<213>    Artificial Sequence

<220>
<223>    FR2 (frame region between CDR-H1 and CDR-H2 of VH-hu1)

<400>    30
Trp Ile Arg Gln Pro Pro Gly Lys Gly Leu Glu Trp Ile Gly
1               5                   10

<210>    31
<211>    14
<212>    PRT
<213>    Artificial Sequence

<220>
<223>    FR2 (frame region between CDR-H1 and CDR-H2 of VH-hu2)

<400>    31
Trp Ile Arg Gln Pro Pro Gly Lys Gly Leu Glu Trp Met Gly
1               5                   10

<210>    32
<211>    14
<212>    PRT
<213>    Artificial Sequence

<220>
<223>    FR2 (frame region between CDR-H1 and CDR-H2 of VH-hu5)

<400>    32
Trp Ile Arg Gln Pro Pro Gly Lys Gly Leu Glu Trp Ile Gly
1               5                   10

<210>    33
<211>    14
<212>    PRT
<213>    Artificial Sequence

<220>
<223>    FR2 (frame region between CDR-H1 and CDR-H2 of VH-hu3)

<400>    33
Trp Val Arg Gln Ala Pro Gly Lys Gly Leu Glu Trp Met Gly
1               5                   10

<210>    34
<211>    32
<212>    PRT
<213>    Artificial Sequence

<220>
<223>    FR3 (frame region between CDR-H2 and CDR-H3 of 10D6)


<400>    34
Arg Ser Ser Ile Thr Arg Asp Thr Ser Lys Asn Gln Phe Phe Leu Gln
1               5                   10                  15

Leu Asn Ser Val Thr Thr Gly Asp Thr Ala Thr Tyr Tyr Cys Ala Arg
            20                  25                  30




<210>    35
<211>    32
<212>    PRT
<213>    Artificial Sequence

<220>
<223>    FR3 (frame region between CDR-H2 and CDR-H3 of VH-hu1)


<400>    35
Arg Val Thr Ile Ser Val Asp Thr Ser Lys Asn Gln Phe Ser Leu Lys
1               5                   10                  15

Leu Ser Ser Val Thr Ala Ala Asp Thr Ala Val Tyr Tyr Cys Ala Arg
            20                  25                  30




<210>    36
<211>    32
<212>    PRT
<213>    Artificial Sequence

<220>
<223>    FR3 (frame region between CDR-H2 and CDR-H3 of VH-hu2)


<400>    36
Arg Ser Thr Ile Ser Arg Asp Thr Ser Lys Asn Gln Phe Ser Leu Lys
1               5                   10                  15

Leu Ser Ser Val Thr Ala Ala Asp Thr Ala Val Tyr Tyr Cys Ala Arg
            20                  25                  30




<210>    37
<211>    32
<212>    PRT
<213>    Artificial Sequence

<220>
<223>    FR3 (frame region between CDR-H2 and CDR-H3 of VH-hu5)


<400>    37
Arg Val Thr Ile Ser Val Asp Thr Ser Lys Asn Gln Phe Ser Leu Lys
1               5                   10                  15

Leu Ser Ser Val Thr Ala Ala Asp Thr Ala Val Tyr Tyr Cys Ala Arg
            20                  25                  30




<210>    38
<211>    32
<212>    PRT
<213>    Artificial Sequence

<220>
<223>    FR3 (frame region between CDR-H2 and CDR-H3 of VH-hu3)


<400>    38
Arg Ser Thr Ile Ser Arg Asp Thr Ser Lys Asn Thr Phe Tyr Leu Gln
1               5                   10                  15

Met Asn Ser Leu Arg Ala Glu Asp Thr Ala Val Tyr Tyr Cys Ala Arg
            20                  25                  30




<210>    39
<211>    11
<212>    PRT
<213>    Artificial Sequence

<220>
<223>    FR4 (C-terminal frame region of CDR-H3 of 10D6)


<400>    39
Trp Gly Gln Gly Thr Ser Val Thr Val Ser Ser
1               5                   10


<210>    40
<211>    11
<212>    PRT
<213>    Artificial Sequence

<220>
<223>    FR4 (C-terminal frame region of CDR-H3 of VH-hu1)


<400>    40
Trp Gly Gln Gly Thr Leu Val Thr Val Ser Ser
1               5                   10


<210>    41
<211>    11

<212>     PRT
<213>     Artificial Sequence

<220>
<223>     FR4 (C-terminal frame region of CDR-H3 of VH-hu2)


<400>     41
Trp Gly Gln Gly Thr Leu Val Thr Val Ser Ser
1               5                   10


<210>     42
<211>     11
<212>     PRT
<213>     Artificial Sequence

<220>
<223>     FR4 (C-terminal frame region of CDR-H3 of VH-hu5)


<400>     42
Trp Gly Gln Gly Thr Leu Val Thr Val Ser Ser
1               5                   10


<210>     43
<211>     11
<212>     PRT
<213>     Artificial Sequence

<220>
<223>     FR4 (C-terminal frame region of CDR-H3 of VH-hu3)


<400>     43
Trp Gly Gln Gly Thr Leu Val Thr Val Ser Ser
1               5                   10


<210>     44
<211>     23
<212>     PRT
<213>     Artificial Sequence

<220>
<223>     FR1 (N-terminal frame region of CDR-L1 of 10D6)


<400>     44
Ser Ile Val Met Thr Gln Thr Pro Lys Phe Leu Leu Val Ser Ala Gly
1               5                   10                  15

Asp Arg Val Thr Ile Thr Cys
            20


<210>     45
<211>     23
<212>     PRT
<213>     Artificial Sequence

<220>

<223>     FR1 (N-terminal frame region of CDR-L1 of VL-hu1)

<400>     45
Asp Ile Gln Met Thr Gln Ser Pro Ser Ser Leu Ser Ala Ser Val Gly
1               5                   10                  15

Asp Arg Val Thr Ile Thr Cys
            20

<210>     46
<211>     15
<212>     PRT
<213>     Artificial Sequence

<220>
<223>     FR2 (frame region between CDR-L1 and CDR-L2 of 10D6)

<400>     46
Trp Tyr Gln Gln Lys Pro Gly Gln Ser Pro Lys Leu Leu Ile Tyr
1               5                   10                  15

<210>     47
<211>     15
<212>     PRT
<213>     Artificial Sequence

<220>
<223>     FR2 (frame region between CDR-L1 and CDR-L2 of VL-hu1)

<400>     47
Trp Tyr Gln Gln Lys Pro Gly Lys Ala Pro Lys Leu Leu Ile Tyr
1               5                   10                  15

<210>     48
<211>     32
<212>     PRT
<213>     Artificial Sequence

<220>
<223>     FR3 (frame region between CDR-L2 and CDR-L3 of 10D6)

<400>     48
Gly Val Pro Asp Arg Phe Thr Gly Ser Gly Tyr Gly Thr Asp Phe Thr
1               5                   10                  15

Phe Thr Ile Ser Thr Val Gln Ala Glu Asp Leu Ala Val Tyr Phe Cys
            20                  25                  30

<210>     49
<211>     32
<212>     PRT
<213>     Artificial Sequence

<220>
<223>    FR3 (frame region between CDR-L2 and CDR-L3 of VL-hu1)

<400>    49
Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>    50
<211>    10
<212>    PRT
<213>    Artificial Sequence

<220>
<223>    FR4 (C-terminal frame region of CDR-L3 of 10D6)

<400>    50
Phe Gly Gly Gly Thr Lys Leu Glu Ile Lys
1               5                   10

<210>    51
<211>    10
<212>    PRT
<213>    Artificial Sequence

<220>
<223>    FR4 (C-terminal frame region of CDR-L3 of VL-hu1)

<400>    51
Phe Gly Gln Gly Thr Lys Val Glu Ile Lys
1               5                   10

<210>    52
<211>    118
<212>    PRT
<213>    Artificial Sequence

<220>
<223>    Heavy chain variable region

<400>    52
Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Thr Ser Asp
            20                  25                  30

Tyr Ala Trp Asn Trp Ile Arg Gln Pro Pro Gly Lys Gly Leu Glu Trp
        35                  40                  45

Met Gly Lys Ile Ser Tyr Ser Gly Lys Thr Asp Tyr Asn Pro Ser Leu
    50                  55                  60

Lys Ser Arg Ser Thr Ile Ser Arg Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Asn Phe Glu Gly Ala Met Asp Tyr Trp Gly Gln Gly Thr
            100                 105                 110

Leu Val Thr Val Ser Ser
        115


<210>   53
<211>   118
<212>   PRT
<213>   Artificial Sequence

<220>
<223>   Heavy chain variable region


<400>   53
Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Thr Ser Asp
            20                  25                  30

Tyr Ala Trp Asn Trp Ile Arg Gln Pro Pro Gly Lys Gly Leu Glu Trp
        35                  40                  45

Met Gly Lys Ile Asn Tyr Ala Gly Asn Thr Asp Tyr Asn Pro Ser Leu
    50                  55                  60

Lys Ser Arg Ser Thr Ile Ser Arg Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Asn Phe Glu Gly Ala Met Asp Tyr Trp Gly Gln Gly Thr
            100                 105                 110

Leu Val Thr Val Ser Ser
        115


<210>   54
<211>   118
<212>   PRT
<213>   Artificial Sequence

<220>
<223>   Heavy chain variable region


<400>   54
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Ile Thr Ser Asp

                    20                  25                  30

Tyr Ala Trp Asn Trp Val Arg Gln Ala Pro Gly Lys Gly Leu Glu Trp
        35                  40                  45

Met Gly Lys Ile Ser Tyr Ser Gly Lys Thr Asp Tyr Asn Pro Ser Leu
    50                  55                  60

Lys Ser Arg Ser Thr Ile Ser Arg Asp Thr Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Asn Phe Glu Gly Ala Met Asp Tyr Trp Gly Gln Gly Thr
            100                 105                 110

Leu Val Thr Val Ser Ser
        115


<210>   55
<211>   118
<212>   PRT
<213>   Artificial Sequence

<220>
<223>   Heavy chain variable region


<400>   55
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Ile Thr Ser Asp
            20                  25                  30

Tyr Ala Trp Asn Trp Val Arg Gln Ala Pro Gly Lys Gly Leu Glu Trp
        35                  40                  45

Met Gly Lys Ile Asn Tyr Ala Gly Asn Thr Asp Tyr Asn Pro Ser Leu
    50                  55                  60

Lys Ser Arg Ser Thr Ile Ser Arg Asp Thr Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Asn Phe Glu Gly Ala Met Asp Tyr Trp Gly Gln Gly Thr
            100                 105                 110

Leu Val Thr Val Ser Ser
        115


<210>   56
<211>   118
<212>   PRT
<213>   Artificial Sequence

<220>
<223>   Heavy chain variable region

<400>    56
Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Thr Ser Asp
            20                  25                  30

Tyr Ala Trp Asn Trp Ile Arg Gln Pro Pro Gly Lys Gly Leu Glu Trp
        35                  40                  45

Ile Gly Tyr Ile Asn Tyr Ser Gly Asn Thr Asp Tyr Asn Pro Ser Leu
    50                  55                  60

Lys Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Asn Phe Glu Gly Ala Met Asp Tyr Trp Gly Gln Gly Thr
            100                 105                 110

Leu Val Thr Val Ser Ser
        115


<210>    57
<211>    107
<212>    PRT
<213>    Artificial Sequence

<220>
<223>    Light chain variable region


<400>    57
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Ser Val Ser Asn Asp
            20                  25                  30

Val Ala Trp Tyr Gln Gln Lys Pro Gly Lys Ala Pro Lys Leu Leu Ile
        35                  40                  45

Tyr Tyr Ala Ser Asn Arg Tyr Pro Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asp Tyr Ser Ser Pro Trp
                85                  90                  95

Thr Phe Gly Gln Gly Thr Lys Leu Glu Ile Lys
            100                 105


<210>    58
<211>    107
<212>    PRT

<213>    Artificial Sequence

<220>
<223>    Light chain variable region


<400>    58
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Ser Val Ser Asn Asp
            20                  25                  30

Val His Trp Tyr Gln Gln Lys Pro Gly Lys Ala Pro Lys Leu Leu Ile
        35                  40                  45

Tyr Tyr Ala Ser Asn Arg Tyr Pro Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asp Tyr Ser Ser Pro Trp
                85                  90                  95

Thr Phe Gly Gln Gly Thr Lys Leu Glu Ile Lys
            100                 105


<210>    59
<211>    107
<212>    PRT
<213>    Artificial Sequence

<220>
<223>    Light chain variable region


<400>    59
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Ser Val Ser Asn Asp
            20                  25                  30

Val His Trp Tyr Gln Gln Lys Pro Gly Lys Ala Pro Lys Leu Leu Ile
        35                  40                  45

Tyr Tyr Ala Ser Asn Arg Tyr Pro Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His Asp Tyr Ser Ser Pro Phe
                85                  90                  95

Thr Phe Gly Gln Gly Thr Lys Leu Glu Ile Lys
            100                 105


<210>    60
<211>    107

<212>    PRT
<213>    Artificial Sequence

<220>
<223>    Light chain variable region


<400>    60
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Phe Val Ser Thr Asp
            20                  25                  30

Val His Trp Tyr Gln Gln Lys Pro Gly Lys Ala Pro Lys Leu Leu Ile
        35                  40                  45

Tyr Tyr Ala Ser Asn Arg Tyr Pro Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asp Tyr Ser Ser Pro Trp
                85                  90                  95

Thr Phe Gly Gln Gly Thr Lys Leu Glu Ile Lys
            100                 105


<210>    61
<211>    107
<212>    PRT
<213>    Artificial Sequence

<220>
<223>    Light chain variable region


<400>    61
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Ser Val Ser Asn Asp
            20                  25                  30

Val Ala Trp Tyr Gln Gln Lys Pro Gly Lys Ala Pro Lys Leu Leu Ile
        35                  40                  45

Tyr Tyr Ala Ser Ile Pro Tyr Pro Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asp Tyr Ser Ser Pro Trp
                85                  90                  95

Thr Phe Gly Gln Gly Thr Lys Leu Glu Ile Lys
            100                 105


<210>    62

<211>    107
<212>    PRT
<213>    Artificial Sequence

<220>
<223>    Light chain variable region


<400>    62
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Ser Val Ser Asn Asp
            20                  25                  30

Val His Trp Tyr Gln Gln Lys Pro Gly Lys Ala Pro Lys Leu Leu Ile
        35                  40                  45

Tyr Tyr Ala Ser Ile Pro Tyr Pro Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asp Tyr Ser Ser Pro Trp
                85                  90                  95

Thr Phe Gly Gln Gly Thr Lys Leu Glu Ile Lys
            100                 105


<210>    63
<211>    107
<212>    PRT
<213>    Artificial Sequence

<220>
<223>    Light chain variable region


<400>    63
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Phe Val Ser Thr Asp
            20                  25                  30

Val His Trp Tyr Gln Gln Lys Pro Gly Lys Ala Pro Lys Leu Leu Ile
        35                  40                  45

Tyr Tyr Ala Ser Ile Pro Tyr Pro Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asp Tyr Ser Ser Pro Trp
                85                  90                  95

Thr Phe Gly Gln Gly Thr Lys Leu Glu Ile Lys
            100                 105

<210>    64
<211>    354
<212>    DNA
<213>    Artificial Sequence

<220>
<223>    Coding sequence of heavy chain variable region(SEQ ID NO: 52)


<400>    64
caggtgcaac tgcaggagtc aggccccggc ctggtaaaac cttctgaaac gctctcactt      60

acctgtgccg ttagtggata ctctatcact tccgactacg cttggaattg gattcggcag      120

cctccaggca aagggctgga atggatggga aagatttcct attccggtaa gactgactac      180

aatcccagtc tgaagagcag gtcaacaatc tccagagaca ccagcaagaa tcagttttcc      240

ctgaaattgt cctcggtgac agcagcggat accgcagtgt attattgcgc ccgcggtaac      300

ttcgagggag ctatggatta ctgggggcag ggtactctcg tcactgtgag cagc      354


<210>    65
<211>    354
<212>    DNA
<213>    Artificial Sequence

<220>
<223>    Coding sequence of heavy chain variable region(SEQ ID NO: 53)


<400>    65
caggtgcaac tgcaggagtc aggccccggc ctggtaaaac cttctgaaac gctctcactt      60

acctgtgccg ttagtggata ctctatcact tccgactacg cttggaattg gattcggcag      120

cctccaggca aagggctgga atggatggga aagattaact atgccggtaa cactgactac      180

aatcccagtc tgaagagcag gtcaacaatc tccagagaca ccagcaagaa tcagttttcc      240

ctgaaattgt cctcggtgac agcagcggat accgcagtgt attattgcgc ccgcggtaac      300

ttcgagggag ctatggatta ctgggggcag ggtactctcg tcactgtgag cagc      354


<210>    66
<211>    354
<212>    DNA
<213>    Artificial Sequence

<220>
<223>    Coding sequence of heavy chain variable region(SEQ ID NO: 54)


<400>    66
gaggttcagc tggtcgaaag cggtggggga ctcgtgcagc caggcggttc tcttagatta      60

tcatgtgccg catccgggta ctccatcacc tctgattatg catggaactg ggtcagacaa      120

gcccccggaa agggcctgga gtggatgggg aagatctcct attcagggaa gacagattat      180

aatccttcgc tgaaaagcag atcaacaatt agtagagaca cttctaaaaa tactttttac      240

ctccagatga acagtctgcg cgccgaagac accgccgtgt actactgcgc taggggaaat 300

ttcgagggag ctatggacta ttggggccag ggcacgttgg taaccgtgag cagc 354

<210> 67
<211> 354
<212> DNA
<213> Artificial Sequence

<220>
<223> Coding sequence of heavy chain variable region(SEQ ID NO: 55)

<400> 67
gaggttcaac tggtagagtc cgggggcggc ctggtccagc caggaggaag cctgcggctc 60

tcttgtgccg ccagcgggta tagtatcact tcagattatg cctggaattg ggtccgccag 120

gcccccggga agggcttaga gtggatgggt aaaattaatt acgcaggcaa caccgactat 180

aatccttcac tgaaatctag atccaccatc tctagagata caagtaagaa caccttttac 240

ttgcagatga atagcctcag ggctgaagac actgctgtgt actactgcgc aagaggaaac 300

ttcgaaggag cgatggatta ttggggccag ggtacgcttg tgacagtgtc ctct 354

<210> 68
<211> 354
<212> DNA
<213> Artificial Sequence

<220>
<223> Coding sequence of heavy chain variable region(SEQ ID NO: 56)

<400> 68
caggtgcagc tgcaggagtc gggcccagga ctggtgaagc cttcggagac cctgtccctc 60

acctgcgctg tctctggtta ctccatcacc agtgattatg cctggaactg gatccggcag 120

cccccaggga aggggctgga gtggattggg tacataaact acagtggtaa cactgactac 180

aacccatctc tcaaaagtcg agtcaccata tcagtagaca cgtccaagaa ccagttctcc 240

ctgaagctga gctctgtgac cgccgcagac acggccgtgt attactgtgc gagaggtaac 300

ttcgaaggtg ctatggacta ctggggtcaa ggaacgcttg tgacagtgtc ctct 354

<210> 69
<211> 321
<212> DNA
<213> Artificial Sequence

<220>
<223> Coding sequence of light chain variable region(SEQ ID NO: 57)

<400> 69
gacatccaga tgacccagtc tccatcctcc ctgtctgcat ctgtaggaga cagagtcacc 60

atcacttgca aggccagtca gagtgtgagt aatgatgtag cttggtatca gcagaaacca 120

gggaaagccc ctaagctcct gatctattat gcatccaatc gctaccctgg ggtcccatca 180

aggttcagtg gcagtggatc tgggacagat ttcactctca ccatcagcag tctgcaacct 240

gaagattttg caacttacta ctgtcagcag gattatagct ctccgtggac gttcggtgga 300

ggcaccaagg tggaaatcaa a 321

<210> 70
<211> 321
<212> DNA
<213> Artificial Sequence

<220>
<223> Coding sequence of light chain variable region(SEQ ID NO: 58)

<400> 70
gacatccaga tgacccagtc tccatcctcc ctgtctgcat ctgtaggaga cagagtcacc 60

atcacttgca aggccagtca gagtgtgagt aatgatgtac attggtatca gcagaaacca 120

gggaaagccc ctaagctcct gatctattat gcatccaatc gctaccctgg ggtcccatca 180

aggttcagtg gcagtggatc tgggacagat ttcactctca ccatcagcag tctgcaacct 240

gaagattttg caacttacta ctgtcagcag gattatagct ctccgtggac gttcggtgga 300

ggcaccaagg tggaaatcaa a 321

<210> 71
<211> 321
<212> DNA
<213> Artificial Sequence

<220>
<223> Coding sequence of light chain variable region(SEQ ID NO: 59)

<400> 71
gacatccaga tgacccagtc tccatcctcc ctgtctgcat ctgtaggaga cagagtcacc 60

atcacttgca aggccagtca gagtgtgagt aatgatgtac attggtatca gcagaaacca 120

gggaaagccc ctaagctcct gatctattat gcatccaatc gctaccctgg ggtcccatca 180

aggttcagtg gcagtggatc tgggacagat ttcactctca ccatcagcag tctgcaacct 240

gaagattttg caacttacta ctgtcagcat gattatagct ctccgttcac gttcggtgga 300

ggcaccaagg tggaaatcaa a 321

<210> 72
<211> 321
<212> DNA
<213> Artificial Sequence

<220>
<223>    Coding sequence of light chain variable region(SEQ ID NO: 60)

<400>    72
gacatccaga tgacccagtc tccatcctcc ctgtctgcat ctgtaggaga cagagtcacc      60

atcacttgca aggccagtca gttcgtgagt actgatgtac attggtatca gcagaaacca     120

gggaaagccc ctaagctcct gatctattat gcatccaatc gctaccctgg ggtcccatca     180

aggttcagtg gcagtggatc tgggacagat ttcactctca ccatcagcag tctgcaacct     240

gaagattttg caacttacta ctgtcagcag gattatagct ctccgtggac gttcggtgga     300

ggcaccaagg tggaaatcaa a                                               321

<210>    73
<211>    321
<212>    DNA
<213>    Artificial Sequence

<220>
<223>    Coding sequence of light chain variable region(SEQ ID NO: 61)

<400>    73
gacatccaga tgacccagtc tccatcctcc ctgtctgcat ctgtaggaga cagagtcacc      60

atcacttgca aggccagtca gagtgtgagt aatgatgtag cttggtatca gcagaaacca     120

gggaaagccc ctaagctcct gatctattat gcatccatcc cataccctgg ggtcccatca     180

aggttcagtg gcagtggatc tgggacagat ttcactctca ccatcagcag tctgcaacct     240

gaagattttg caacttacta ctgtcagcag gattatagct ctccgtggac gttcggtgga     300

ggcaccaagg tggaaatcaa a                                               321

<210>    74
<211>    321
<212>    DNA
<213>    Artificial Sequence

<220>
<223>    Coding sequence of light chain variable region(SEQ ID NO: 62)

<400>    74
gacatccaga tgacccagtc tccatcctcc ctgtctgcat ctgtaggaga cagagtcacc      60

atcacttgca aggccagtca gagtgtgagt aatgatgtac attggtatca gcagaaacca     120

gggaaagccc ctaagctcct gatctattat gcatccatcc cataccctgg ggtcccatca     180

aggttcagtg gcagtggatc tgggacagat ttcactctca ccatcagcag tctgcaacct     240

gaagattttg caacttacta ctgtcagcag gattatagct ctccgtggac gttcggtgga     300

ggcaccaagg tggaaatcaa a                                               321

<210>    75
<211>    321
<212>    DNA
<213>    Artificial Sequence

<220>
<223>    Coding sequence of light chain variable region(SEQ ID NO: 63)


<400>    75
gacatccaga tgacccagtc tccatcctcc ctgtctgcat ctgtaggaga cagagtcacc        60

atcacttgca aggccagtca gttcgtgagt actgatgtac attggtatca gcagaaacca       120

gggaaagccc ctaagctcct gatctattat gcatccatcc cataccctgg ggtcccatca       180

aggttcagtg gcagtggatc tgggacagat ttcactctca ccatcagcag tctgcaacct       240

gaagattttg caacttacta ctgtcagcag gattatagct ctccgtggac gttcggtgga       300

ggcaccaagg tggaaatcaa a                                                 321


<210>    76
<211>    15
<212>    PRT
<213>    Artificial Sequence

<220>
<223>    Linker


<400>    76
Gly Gly Gly Gly Ser Gly Gly Gly Gly Ser Gly Gly Gly Gly Ser
1               5                   10                  15


<210>    77
<211>    118
<212>    PRT
<213>    Artificial Sequence

<220>
<223>    10D6-HU1


<400>    77
Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Ser Ser Asp
            20                  25                  30

Tyr Ala Trp Asn Trp Ile Arg Gln Pro Pro Gly Lys Gly Leu Glu Trp
        35                  40                  45

Ile Gly Tyr Ile Asn Tyr Ser Gly Asn Thr Asp Tyr Asn Pro Ser Leu
    50                  55                  60

Lys Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Asn Phe Glu Gly Ala Met Asp Tyr Trp Gly Gln Gly Thr
            100                 105                 110

Leu Val Thr Val Ser Ser
        115

<210>   78
<211>   118
<212>   PRT
<213>   Artificial Sequence

<220>
<223>   10D6-HU2

<400>   78
Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Thr Ser Asp
            20                  25                  30

Tyr Ala Trp Asn Trp Ile Arg Gln Pro Pro Gly Lys Gly Leu Glu Trp
        35                  40                  45

Met Gly Tyr Ile Asn Tyr Ser Gly Asn Thr Asp Tyr Asn Pro Ser Leu
    50                  55                  60

Lys Ser Arg Ser Thr Ile Ser Arg Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Asn Phe Glu Gly Ala Met Asp Tyr Trp Gly Gln Gly Thr
            100                 105                 110

Leu Val Thr Val Ser Ser
        115

<210>   79
<211>   118
<212>   PRT
<213>   Artificial Sequence

<220>
<223>   10D6-HU3

<400>   79
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Ile Thr Ser Asp
            20                  25                  30

Tyr Ala Trp Asn Trp Val Arg Gln Ala Pro Gly Lys Gly Leu Glu Trp
        35                  40                  45

Met Gly Tyr Ile Asn Tyr Ser Gly Asn Thr Asp Tyr Asn Pro Ser Leu
    50                  55                  60

Lys Ser Arg Ser Thr Ile Ser Arg Asp Thr Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Asn Phe Glu Gly Ala Met Asp Tyr Trp Gly Gln Gly Thr
            100                 105                 110

Leu Val Thr Val Ser Ser
        115

<210>    80
<211>    240
<212>    PRT
<213>    Artificial Sequence

<220>
<223>    VH-linker-VL (scFv) of 10D6

<400>    80
Asp Val Gln Leu Gln Glu Ser Gly Pro Asp Leu Val Lys Pro Ser Gln
1               5                   10                  15

Ser Leu Ser Leu Thr Cys Thr Val Thr Gly Tyr Ser Ile Thr Ser Asp
            20                  25                  30

Tyr Ala Trp Asn Trp Ile Arg Gln Phe Pro Gly Asn Lys Leu Glu Trp
        35                  40                  45

Met Gly Tyr Ile Asn Tyr Ser Gly Asn Thr Asp Tyr Asn Pro Ser Leu
    50                  55                  60

Lys Ser Arg Ser Ser Ile Thr Arg Asp Thr Ser Lys Asn Gln Phe Phe
65                  70                  75                  80

Leu Gln Leu Asn Ser Val Thr Thr Gly Asp Thr Ala Thr Tyr Tyr Cys
                85                  90                  95

Ala Arg Gly Asn Phe Glu Gly Ala Met Asp Tyr Trp Gly Gln Gly Thr
            100                 105                 110

Leu Val Thr Val Ser Ser Gly Gly Gly Gly Ser Gly Gly Gly Gly Ser
        115                 120                 125

Gly Gly Gly Gly Ser Ser Ile Val Met Thr Gln Thr Pro Lys Phe Leu
    130                 135                 140

Leu Val Ser Ala Gly Asp Arg Val Thr Ile Thr Cys Lys Ala Ser Gln
145                 150                 155                 160

Ser Val Ser Asn Asp Val Ala Trp Tyr Gln Gln Lys Pro Gly Gln Ser
                165                 170                 175

Pro Lys Leu Leu Ile Tyr Tyr Ala Ser Asn Arg Tyr Pro Gly Val Pro
            180                 185                 190

Asp Arg Phe Thr Gly Ser Gly Tyr Gly Thr Asp Phe Thr Phe Thr Ile
        195                 200                 205

Ser Thr Val Gln Ala Glu Asp Leu Ala Val Tyr Phe Cys Gln Gln Asp
    210                 215                 220

Tyr Ser Ser Pro Trp Thr Phe Gly Gly Gly Thr Lys Leu Glu Ile Lys
225                 230                 235                 240

<210> 81
<211> 720
<212> DNA
<213> Artificial Sequence

<220>
<223> Coding sequence of VH-linker-VL (scFv) of 10D6

<400> 81
gatgtgcagc ttcaggagtc gggacctgac ctggtgaaac cttctcagtc tctgtccctc 60

acctgcactg tcactggcta ctcaatcacc agtgattatg cctggaactg gatccggcag 120

tttccaggaa acaaactgga gtggatgggc tacataaact acagtggtaa cactgactac 180

aacccatctc tcaaaagtcg aagctctatc actcgagaca catccaagaa ccagttcttc 240

ctgcagttga attctgtgac tactggggac acagccacat attactgtgc aagaggtaac 300

ttcgaaggtg ctatggacta ctggggtcaa ggaacctcag tcaccgtctc ctcaggcggc 360

ggcggcagcg gcggcggcgg cagcggcggc ggcggcagca gtattgtgat gacccagact 420

cccaaattcc tgcttgtatc agcaggagac agggttacca taacctgcaa ggccagtcag 480

agtgtgagta atgatgtagc ttggtaccaa cagaagccag ggcagtctcc taaactgctg 540

atatactatg catccaatcg ctaccctgga gtccctgatc gcttcactgg cagtggatat 600

gggacggatt tcactttcac catcagcact gtgcaggctg aagacctggc agtttatttc 660

tgtcagcagg attatagctc tccgtggacg ttcggtggag gcaccaagct ggaaatcaaa 720

720

<210> 82
<211> 240
<212> PRT
<213> Artificial Sequence

<220>
<223> VH-linker-VL (scFv) of10D6 opti-1

<400> 82
Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Thr Ser Asp
            20                  25                  30

Tyr Ala Trp Asn Trp Ile Arg Gln Pro Pro Gly Lys Gly Leu Glu Trp
        35                  40                  45

Met Gly Lys Ile Ser Tyr Ser Gly Lys Thr Asp Tyr Asn Pro Ser Leu
    50                  55                  60

Lys Ser Arg Ser Thr Ile Ser Arg Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Asn Phe Glu Gly Ala Met Asp Tyr Trp Gly Gln Gly Thr
            100                 105                 110

Leu Val Thr Val Ser Ser Gly Gly Gly Gly Ser Gly Gly Gly Gly Ser
        115                 120                 125

Gly Gly Gly Gly Ser Asp Ile Gln Met Thr Gln Ser Pro Ser Ser Leu
    130                 135                 140

Ser Ala Ser Val Gly Asp Arg Val Thr Ile Thr Cys Lys Ala Ser Gln
145                 150                 155                 160

Ser Val Ser Asn Asp Val Ala Trp Tyr Gln Gln Lys Pro Gly Lys Ala
                165                 170                 175

Pro Lys Leu Leu Ile Tyr Tyr Ala Ser Asn Arg Tyr Pro Gly Val Pro
            180                 185                 190

Ser Arg Phe Ser Gly Ser Gly Ser Gly Thr Asp Phe Thr Leu Thr Ile
        195                 200                 205

Ser Ser Leu Gln Pro Glu Asp Phe Ala Thr Tyr Tyr Cys Gln Gln Asp
    210                 215                 220

Tyr Ser Ser Pro Trp Thr Phe Gly Gln Gly Thr Lys Leu Glu Ile Lys
225                 230                 235                 240


<210>   83
<211>   720
<212>   DNA
<213>   Artificial Sequence

<220>
<223>   Coding sequence of VH-linker-VL (scFv) of10D6 opti-1


<400>   83
caggtgcaac tgcaggagtc aggccccggc ctggtaaaac cttctgaaac gctctcactt      60

acctgtgccg ttagtggata ctctatcact tccgactacg cttggaattg gattcggcag     120

cctccaggca aagggctgga atggatggga aagatttcct attccggtaa gactgactac     180

aatcccagtc tgaagagcag gtcaacaatc tccagagaca ccagcaagaa tcagttttcc     240

ctgaaattgt cctcggtgac agcagcggat accgcagtgt attattgcgc ccgcggtaac     300

ttcgagggag ctatggatta ctgggggcag ggtactctcg tcactgtgag cagcggcggc     360

ggcggcagcg gcggcggcgg cagcggcggc ggcggcagcg acatccagat gacccagtct     420

ccatcctccc tgtctgcatc tgtaggagac agagtcacca tcacttgcaa ggccagtcag 480

agtgtgagta atgatgtagc ttggtatcag cagaaaccag ggaaagcccc taagctcctg 540

atctattatg catccaatcg ctaccctggg gtcccatcaa ggttcagtgg cagtggatct 600

gggacagatt tcactctcac catcagcagt ctgcaacctg aagattttgc aacttactac 660

tgtcagcagg attatagctc tccgtggacg ttcggtggag gcaccaaggt ggaaatcaaa 720

720

<210> 84
<211> 118
<212> PRT
<213> Artificial Sequence

<220>
<223> Amino acid sequence of heavy chain variable region

<400> 84
Asp Val Gln Leu Gln Glu Ser Gly Pro Gly Leu Val Lys Pro Ser Gln
1               5                   10                  15

Ser Leu Ser Leu Thr Cys Thr Val Thr Gly Tyr Ser Ile Thr Ser Asp
            20                  25                  30

Tyr Ala Trp Asn Trp Ile Arg Gln Phe Pro Gly Asn Lys Leu Glu Trp
        35                  40                  45

Met Gly Tyr Ile Asn Tyr Ser Gly Asn Thr Asp Tyr Asn Pro Ser Leu
    50                  55                  60

Lys Ser Arg Ser Ser Ile Thr Arg Asp Thr Ser Lys Asn Gln Phe Phe
65                  70                  75                  80

Leu Gln Leu Asn Ser Val Thr Thr Gly Asp Thr Ala Thr Tyr Tyr Cys
                85                  90                  95

Ala Arg Gly Asn Phe Glu Gly Ala Met Asp Tyr Trp Gly Gln Gly Thr
            100                 105                 110

Leu Val Thr Val Ser Ser
        115

<210> 85
<211> 30
<212> PRT
<213> Artificial Sequence

<220>
<223> FR1 (N-terminal frame region of CDR-H1 of 10D6)

<400> 85
Asp Val Gln Leu Gln Glu Ser Gly Pro Gly Leu Val Lys Pro Ser Gln
1               5                   10                  15

Ser Leu Ser Leu Thr Cys Thr Val Thr Gly Tyr Ser Ile Thr

20                  25                  30

<210>   86
<211>   11
<212>   PRT
<213>   Artificial Sequence

<220>
<223>   FR4 (C-terminal frame region of CDR-H3 of 10D6)

<400>   86
Trp Gly Gln Gly Thr Leu Val Thr Val Ser Ser
1               5                   10

<210>   87
<211>   107
<212>   PRT
<213>   Artificial Sequence

<220>
<223>   Light chain variable region

<400>   87
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Ser Val Ser Asn Asp
            20                  25                  30

Val Ala Trp Tyr Gln Gln Lys Pro Gly Lys Ala Pro Lys Leu Leu Ile
        35                  40                  45

Tyr Tyr Ala Ser Asn Arg Tyr Pro Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gly Gln Asp Tyr Ala Ser Pro Trp
                85                  90                  95

Thr Phe Gly Gln Gly Thr Lys Leu Glu Ile Lys
            100                 105

<210>   88
<211>   321
<212>   DNA
<213>   Artificial Sequence

<220>
<223>   Nucleotide sequence coding VL (h10D6-OPTI-64)

<400>   88
gacatccaga tgacccagtc tccatcctcc ctgtctgcat ctgtaggaga cagagtcacc      60

atcacttgca aggccagtca gagtgtgagt aatgatgtag cttggtatca gcagaaacca      120

gggaaagccc ctaagctcct gatctattat gcatccaacc gataccctgg ggtcccatca 180

aggttcagtg gcagtggatc tgggacagat ttcactctca ccatcagcag tctgcaacct 240

gaagattttg caacttacta ctgtggacag gattatgcct ctccgtggac gttcggtgga 300

ggcaccaagg tggaaatcaa a 321

<210> 89
<211> 107
<212> PRT
<213> Artificial Sequence

<220>
<223> Light chain variable region

<400> 89
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Ser Val Ser Asn Asp
            20                  25                  30

Val His Trp Tyr Gln Gln Lys Pro Gly Lys Ala Pro Lys Leu Leu Ile
        35                  40                  45

Tyr Tyr Ala Ser Asn Arg Tyr Pro Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gly Gln Asp Tyr Ser Ala Pro Trp
                85                  90                  95

Thr Phe Gly Gln Gly Thr Lys Leu Glu Ile Lys
            100                 105

<210> 90
<211> 321
<212> DNA
<213> Artificial Sequence

<220>
<223> Nucleotide sequecne coding VL (h10D6-OPTI-66)

<400> 90
gacatccaga tgacccagtc tccatcctcc ctgtctgcat ctgtaggaga cagagtcacc 60

atcacttgca aggccagtca gagtgtgagt aatgatgtac actggtatca gcagaaacca 120

gggaaagccc ctaagctcct gatctattat gcatccaacc gataccctgg ggtcccatca 180

aggttcagtg gcagtggatc tgggacagat ttcactctca ccatcagcag tctgcaacct 240

gaagattttg caacttacta ctgtggacag gattattctg ccccgtggac gttcggtgga 300

ggcaccaagg tggaaatcaa a 321